(19) Europäisches Patentamt
European Patent Office
Office européen des brevets

(11) **EP 2 928 849 B1**

(12) **EUROPÄISCHE PATENTSCHRIFT**

(45) Veröffentlichungstag und Bekanntmachung des Hinweises auf die Patenterteilung:
**22.02.2017 Patentblatt 2017/08**

(21) Anmeldenummer: **13802325.4**

(22) Anmeldetag: **04.12.2013**

(51) Int Cl.:
**C07C 5/48** (2006.01)

(86) Internationale Anmeldenummer:
**PCT/EP2013/075456**

(87) Internationale Veröffentlichungsnummer:
**WO 2014/086815 (12.06.2014 Gazette 2014/24)**

(54) **VERFAHREN ZUR OXIDATIVEN DEHYDRIERUNG VON N-BUTENEN ZU BUTADIEN**

METHOD FOR THE OXIDATIVE DEHYDROGENATION OF N-BUTENES INTO BUTADIENE

PROCÉDÉ DE DÉSHYDROGÉNATION OXYDATIVE DE N-BUTÈNES EN BUTADIÈNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.12.2012 EP 12195858**

(43) Veröffentlichungstag der Anmeldung:
**14.10.2015 Patentblatt 2015/42**

(73) Patentinhaber: **BASF SE**
**67056 Ludwigshafen am Rhein (DE)**

(72) Erfinder:
• **GRÜNE, Philipp**
**68161 Mannheim (DE)**
• **RÜTTINGER, Wolfgang**
**68161 Mannheim (DE)**
• **WALSDORFF, Christian**
**67061 Ludwigshafen (DE)**

(56) Entgegenhaltungen:
**WO-A2-2009/124945    JP-A- 6 058 928
US-A- 3 911 039**

EP 2 928 849 B1

**Beschreibung**

Die Erfindung betrifft ein Verfahren zur oxidativen Dehydrierung von n-Butenen zu Butadien.

Butadien ist eine bedeutende Grundchemikalie und wird beispielsweise zur Herstellung von Synthesekautschuken (Butadien-Homopolymere, Styrol-Butadien-Kautschuk oder Nitril-Kautschuk) oder zur Herstellung von thermoplastischen Terpolymeren (Acrylnitril-Butadien-Styrol-Copolymere) eingesetzt. Butadien wird ferner zu Sulfolan, Chloropren und 1,4-Hexamethylendiamin (über 1,4-Dichlorbuten und Adipinsäuredinitril) umgesetzt. Durch Dimerisierung von Butadien kann ferner Vinylcyclohexen erzeugt werden, welches zu Styrol dehydriert werden kann.

Butadien kann durch thermische Spaltung (Steam-Cracken) gesättigter Kohlenwasserstoffe hergestellt werden, wobei üblicherweise von Naphtha als Rohstoff ausgegangen wird. Beim Steam-Cracken von Naphtha fällt ein Kohlenwasserstoff-Gemisch aus Methan, Ethan, Ethen, Acetylen, Propan, Propen, Propin, Allen, Butanen, Butenen, Butadien, Butinen, Methylallen, $C_5$- und höheren Kohlenwasserstoffen an.

Butadien kann auch durch oxidative Dehydrierung von n-Butenen (1-Buten und/oder 2-Buten) erhalten werden. Als Ausgangsgasgemisch für die oxidative Dehydrierung von n-Butenen zu Butadien kann jedes beliebige n-Butene enthaltende Gemisch benutzt werden. Beispielsweise kann eine Fraktion verwendet werden, die als Hauptbestandteil n-Butene (1-Buten und/oder 2-Buten) enthält und aus der $C_4$-Fraktion eines Naphtha-Crackers durch Abtrennen von Butadien und Isobuten erhalten wurde. Des Weiteren können auch Gasgemische als Ausgangsgas eingesetzt werden, die 1-Buten, cis-2-Buten, trans-2-Buten oder deren Gemische umfassen und durch Dimerisierung von Ethylen erhalten wurden. Ferner können als Ausgangsgas n-Butene enthaltende Gasgemische eingesetzt werden, die durch katalytisches Wirbelschichtcracken (Fluid Catalytic Cracking, FCC) erhalten wurden.

n-Butene enthaltende Gasgemische, die als Ausgangsgas in der oxidativen Dehydrierung von n-Butenen zu Butadien eingesetzt werden, können auch durch nicht-oxidative Dehydrierung von n-Butan enthaltenden Gasgemischen hergestellt werden.

WO2009/124945 offenbart einen Schalenkatalysator für die oxidative Dehydrierung von 1-Buten und/oder 2-Buten zu Butadien, der erhältlich ist aus einem Katalysator-Vorläufer umfassend

(a) einen Trägerköper,

(b) eine Schale enthaltend (i) ein katalytisch aktives, Molybdän und mindestens ein weiteres Metall enthaltendes Multimetalloxid der allgemeinen Formel

$$Mo_{12}Bi_aCr_b\ X^1_cFe_dX^2eX^3_fO_y$$

mit

X$^1$ = Co und/oder Ni,
X$^2$ = Si und/oder Al,
X$^3$ = Li, Na, K, Cs und/oder Rb,

$$0,2 \leq a \leq 1,$$

$$0 \ \leq b \leq 2,$$

$$2 \leq c \leq 10,$$

$$0,5 \leq d \leq 10,$$

$$0 \leq e \leq 10,$$

$$0 \leq f \leq 0,5$$

und y = eine Zahl, die unter der Voraussetzung der Ladungsneutralität durch die Wertigkeit und Häufigkeit der von Sauerstoff verschiedenen Elemente bestimmt wird,

und (ii) mindestens einen Porenbildner.

WO 2010/137595 offenbart einen Multimetalloxidkatalysator für die oxidative Dehydrierung von Alkenen zu Dienen, der zumindest Molybdän, Bismut und Cobalt umfasst, der allgemeinen Formel

$$Mo_aBi_bCo_cNi_dFe_eX_fY_gZ_hSi_iO_j$$

In dieser Formel ist X mindestens ein Element aus der Gruppe bestehend aus Magnesium (Mg), Calcium (Ca), Zink (Zn), Cer (Ce) und Samarium (Sm). Y ist mindestens ein Element aus der Gruppe bestehend aus Natrium (Na), Kalium (K), Rubidium (Rb), Cäsium (Cs) und Thallium (Tl). Z ist mindestens ein Element aus der Gruppe bestehend aus Bor (B), Phosphor (P), Arsen (As) und Wolfram (W). a-j stehen für den Atomanteil des jeweiligen Elements, wobei a=12, b = 0,5-7, c = 0-10, d = 0-10, (wobei c+d = 1-10), e = 0,05-3, f = 0-2, g = 0,04-2, h = 0-3 und i = 5-48 sind. In den Ausführungsbeispielen wird ein Katalysator der Zusammensetzung $Mo_{12}Bi_5Co_{2,5}Ni_{2,5}Fe_{0,4}Na_{0,35}B_{0,2}K_{0,08}Si_{24}$ in Form von Tabletten mit einem Durchmesser von 5 mm und einer Höhe von 4 mm in der oxidativen Dehydrierung von n-Butenen zu Butadien eingesetzt.

Bei der oxidativen Dehydrierung von n-Butenen zu Butadien können Koksvorläufer gebildet werden, beispielsweise Styrol, Anthrachinon und Fluorenon, die schließlich zur Verkokung und Deaktivierung des Multimetalloxid-Katalysators führen können. Durch die Bildung kohlenstoffhaltiger Ablagerungen kann der Druckverlust über dem Katalysatorbett steigen. Es ist möglich zur Regenerierung den auf dem Multimetalloxid-Katalysator abgeschiedenen Kohlenstoff in regelmäßigen Abständen mit einem sauerstoffhaltigen Gas abzubrennen, um die Aktivität des Katalysators wiederherzustellen.

JP 60-058928 beschreibt die Regenerierung eines Multimetalloxid-Katalysators zur oxidativen Dehydrierung von n-Butenen zu Butadien, enthaltend mindestens Molybdän, Bismut, Eisen, Cobalt und Antimon, mit einem sauerstoffhaltigen Gasgemisch bei einer Temperatur von 300 bis 700 °C, vorzugsweise 350 bis 650 °C, und einer Sauerstoffkonzentration von 0,1 bis 5%. Als sauerstoffhaltiges Gasgemisch wird Luft zugeführt, die mit geeigneten Inertgasen wie Stickstoff, Wasserdampf oder Kohlendioxid verdünnt ist.

WO 2005/047226 beschreibt die Regenerierung eines Multimetalloxid-Katalysators zur Partialoxidation von Acrolein zu Acrylsäure, enthaltend mindestens Molybdän und Vanadium, durch Überleiten eines sauerstoffhaltigen Gasgemischs bei einer Temperatur von 200 bis 450 °C. Als sauerstoffhaltiges Gasgemisch wird bevorzugt Magerluft mit 3 bis 10 Vol.-% Sauerstoff eingesetzt. Neben Sauerstoff und Stickstoff kann das Gasgemisch Wasserdampf enthalten.

Aufgabe der Erfindung ist es, ein Verfahren zur oxidativen Dehydrierung von n-Butenen zu Butadien bereitzustellen, bei dem die Regenerierung des Multimetalloxid-Katalysators möglichst wirksam und einfach ist.

Gelöst wird die Aufgabe durch ein Verfahren zur oxidativen Dehydrierung von n-Butenen zu Butadien, umfassend mindestens zwei Produktionsschritte (i) und mindestens einen Regenerierschritt (ii), bei dem

(i) in einem Produktionsschritt ein n-Butene enthaltendes Ausgangsgasgemisch mit einem Sauerstoff enthaltenden Gas gemischt und in einem Festbettreaktor mit einem in einem Katalysatorfestbett angeordneten Multimetalloxid-Katalysator, enthaltend mindestens Molybdän und ein weiteres Metall, in Kontakt gebracht wird, und

(ii) in einem Regenerierschritt der Multimetalloxid-Katalysator durch Überleiten eines sauerstoffhaltigen Regeneriergasgemischs über das Katalysatorfestbett und Abbrennen des auf dem Katalysator abgeschiedenen Kohlenstoffs regeneriert wird,

wobei zwischen zwei Produktionsschritten (i) ein Regenerierschritt (ii) durchgeführt wird,

dadurch gekennzeichnet, dass die mindestens zwei Produktionsschritte (i) bei einer Temperatur von mindestens 350 °C und der mindestens eine Regenerierschritt (ii) bei einer Temperatur durchgeführt wird, die höchstens 50 °C über der Temperatur, bei der der vorausgegangene Produktionsschritt (i) durchgeführt wurde, liegt.

Es wurde gefunden, dass eine Regenerierung des Multimetalloxid-Katalysators bei Temperaturen von mindestens 350 °C dazu führt, dass Aktivität und Selektivität des Multimetalloxid-Katalysators im Wesentlichen erhalten bleiben.

Bevorzugt wird der mindestens eine Regenerierschritt (ii) bei einer Temperatur durchgeführt, die höchstens 20 °C, besonders bevorzugt höchstens 10 °C über der Temperatur, bei der der vorausgegangene Produktionsschritt (i) durchgeführt wurde, liegt. In einer insbesondere bevorzugten Ausführungsform wird der mindestens eine Regenerierschritt (ii) bei der gleichen Temperatur (d. h. innerhalb eines Temperaturfensters von $\pm$ 5 °C) wie der vorausgegangene Produktionsschritt (i) durchgeführt. Bevorzugt ist die Temperaturdifferenz zwischen Produktionsschritten (i) und Regenerierschritten (ii) möglichst gering, um ein energieintensives Aufheizen und Abkühlen des Wärmeaustauschmittels zu vermeiden.

Im Allgemeinen wird auch der Regenerierschritt (ii) bei mindestens 350 °C durchgeführt. Werden die Produktionsschritte (i) bei Temperaturen oberhalb von 350 °C durchgeführt, so werden die Regenerierschritte (ii) im Allgemeinen bei mindestens der gleichen Temperatur wie der jeweils vorausgegangene Produktionsschritt (i) durchgeführt. Sie können auch bei einer um bis zu 20 °C niedrigeren Temperatur, bevorzugt um bis zu 10 °C niedrigeren Temperatur als die jeweils vorausgegangenen Produktionsschritte (i) durchgeführt werden, solange sie bei mindestens 350 °C durchgeführt werden. Die etwas niedrigere Temperatur als im Produktionsschritt kann sich durch Abkühlen des Salzbades in Abwesenheit einer exothermen Reaktion während des Regenerationsschrittes einstellen.

Die Reaktionstemperatur der Oxidehydrierung im Produktionsschritt (i) wird durch ein Wärmeaustauschmittel, welches sich um die Reaktionsrohre herum befindet, kontrolliert. Die Temperatur des Wärmeaustauschmittels entspricht der Reaktionstemperatur und beträgt von 350 bis 490 °C, bevorzugt von 360 bis 450 °C und besonders bevorzugt von 365 bis 420 °C. Im Allgemeinen werden zwei aufeinander folgende Produktionsschritte (i) bei im Wesentlichen der gleichen Temperatur (d. h. innerhalb eines Temperaturfensters von ± 2 °C) durchgeführt. Alle vor- und nachstehend für Produktionsschritt und Regenerationsschritt genannten Temperaturen beziehen sich auf die Temperatur des Wärmeaustauschmediums am Einlass des Wärmeaustauschmediums am Reaktor.

Weiterhin wurde gefunden, dass die Aktivität des Multimetalloxid-Katalysators im Wesentlichen wiederhergestellt wird, wenn nur bis zu 50 Gew.-% des auf dem Katalysator abgeschiedenen Kohlenstoffs abgebrannt werden. Schon nach Abbrand von nur 5 Gew.-% des abgeschiedenen Kohlenstoffs pro Regenerierschritt (ii) wird die Aktivität des Katalysators weitgehend wiederhergestellt. Dadurch, dass pro Regenerationsschritt nur bis zu 50 Gew.-% des abgeschiedenen Kohlenstoffs abgebrannt werden, kann die Regenerationszeit stark verkürzt werden, was die Wirtschaftlichkeit des Verfahrens verbessert.

Die Aktivität des Multimetalloxid-Katalysators wird nach jedem Regenerationsschritt (ii) im Allgemeinen zu über 95%, bevorzugt zu über 98% und insbesondere zu über 99%, bezogen auf die Aktivität des Multimetalloxid-Katalysators zu Beginn des vorhergehenden Produktionsschrittes, wiederhergestellt.

Ein Regenerierschritt (ii) wird im Allgemeinen durchgeführt, wenn der relative Umsatzverlust (also bezogen auf den Umsatz zu Beginn des jeweiligen Produktionsschrittes (i)) bei konstanter Temperatur höchstens 25% beträgt. Vorzugsweise wird ein Regenerierschritt (ii) durchgeführt, bevor der relative Umsatzverlust bei konstanter Temperatur größer 15% ist, insbesondere bevor der relative Umsatzverlust bei konstanter Temperatur größer als 10% ist. Im Allgemeinen wird ein Regenerierschritt (ii) erst dann durchgeführt, wenn der relative Umsatzverlust bei konstanter Temperatur mindestens 2% beträgt.

Im Allgemeinen weist ein Produktionsschritt (i) eine Dauer von 5 bis 5000 h auf, bis ein relativer Umsatzverlust von bis zu 25%, bezogen auf den Umsatz zu Beginn des Produktionsschrittes, erreicht ist. Der Katalysator kann bis zu 5000 oder mehr Zyklen aus Produktions- und Regenerationsschritten durchlaufen.

Die Menge des auf dem Katalysator abgeschiedenen und abgebrannten Kohlenstoffs kann durch quantitative Messung der während des jeweiligen Regenerierschritts (ii) gebildeten Kohlenstoffoxide bestimmt werden, beispielsweise durch online-IR-Bestimmung der Kohlenstoffoxide in den Abgasen der Regenerierung. Die Menge des insgesamt auf dem Katalysator abgeschiedenen Kohlenstoffs wird durch totalen Abbrand des Kohlenstoffs bei mindestens 400 °C mit einem Gemisch aus 10 Vol.-% Sauerstoff, 80 Vol.-% Stickstoff und 10 Vol.-% Wasserdampf bestimmt. Die Temperatur wird dabei so gewählt, dass bei einer weiteren Erhöhung der Temperatur keine zusätzliche Bildung von Kohlenstoffoxiden mehr stattfindet. Alternativ kann die Menge an Kohlenstoffablagerungen auf dem Katalysator durch die Messung des Kohlenstoffgehalts an entnommenen Proben des Katalysators erfolgen.

Für die Oxidehydrierung geeignete Katalysatoren basieren im Allgemeinen auf einem Mo-Bi-O-haltigen Multimetalloxidsystem, das in der Regel zusätzlich Eisen enthält. Im Allgemeinen enthält das Katalysatorsystem noch weitere zusätzliche Komponenten aus der 1. bis 15. Gruppe des Periodensystems, wie beispielsweise Kalium, Cäsium, Magnesium, Zirkon, Chrom, Nickel, Cobalt, Cadmium, Zinn, Blei, Germanium, Lanthan, Mangan, Wolfram, Phosphor, Cer, Aluminium oder Silizium. Auch eisenhaltige Ferrite wurden als Katalysatoren vorgeschlagen.

In einer bevorzugten Ausführungsform enthält das Multimetalloxid Cobalt und/oder Nickel. In einer weiteren bevorzugten Ausführungsform enthält das Multimetalloxid Chrom. In einer weiteren bevorzugten Ausführungsform enthält das Multimetalloxid Mangan.

Im Allgemeinen weist das katalytisch aktive, Molybdän und mindestens ein weiteres Metall enthaltende Multimetalloxid die allgemeinen Formel (I) auf,

$$Mo_{12}Bi_aFe_bCo_cNi_dCr_eX^1_fX^2_gO_x \qquad (I)$$

in der die Variablen nachfolgende Bedeutung aufweisen:

$X^1$ = W, Sn, Mn, La, Ce, Ge, Ti, Zr, Hf, Nb, P, Si, Sb, Al, Cd und/oder Mg;

EP 2 928 849 B1

$X^2$ = Li, Na, K, Cs und/oder Rb,

a = 0,1 bis 7, vorzugsweise 0,3 bis 1,5;

b = 0 bis 5, vorzugsweise 2 bis 4;

c = 0 bis 10, vorzugsweise 3 bis 10;

d = 0 bis 10;

e = 0 bis 5, vorzugsweise 0,1 bis 2;

f = 0 bis 24, vorzugsweise 0,1 bis 2;

g = 0 bis 2, vorzugsweise 0,01 bis 1; und

x = eine Zahl, die durch die Wertigkeit und Häufigkeit der von Sauerstoff verschiedenen Elemente in (I) bestimmt wird.

Der Katalysator kann ein Vollmaterialkatalysator oder ein Schalenkatalysator sein. Falls er ein Schalenkatalysator ist, weist er einen Trägerkörper (a) und eine Schale (b) enthaltend das katalytisch aktive, Molybdän und mindestens ein weiteres Metall enthaltende Multimetalloxid der allgemeinen Formel (I) auf.

Für Schalenkatalysatoren geeignete Trägermaterialien sind z.B. poröse oder bevorzugt unporöse Aluminiumoxide, Siliciumdioxid, Zirkondioxid, Siliciumcarbid oder Silikate wie Magnesium- oder Aluminiumsilicat (z.B. Steatit des Typs C 220 der Fa. CeramTec). Die Materialien der Trägerkörper sind chemisch inert.

Die Trägermaterialien können porös oder nicht porös sein. Vorzugsweise ist das Trägermaterial nicht porös (Gesamtvolumen der Poren auf das Volumen des Trägerkörpers bezogen vorzugsweise $\leq$ 1 Vol.-%).

Besonders geeignet ist die Verwendung von im wesentlichen nicht porösen, oberflächenrauhen, kugelförmigen Trägern aus Steatit (z. B. Steatit des Typs C 220 der Fa. CeramTec), deren Durchmesser 1 bis 8 mm, bevorzugt 2 bis 6 mm, besonders bevorzugt 2 bis 3 oder 4 bis 5 mm beträgt. Geeignet ist aber auch die Verwendung von Zylindern aus chemisch inertem Trägermaterial als Trägerkörper, deren Länge 2 bis 10 mm und deren Außendurchmesser 4 bis 10 mm beträgt. Im Fall von Ringen als Trägerkörper liegt die Wanddicke darüber hinaus üblicherweise bei 1 bis 4 mm. Bevorzugt zu verwendende ringförmige Trägerkörper besitzen eine Länge von 2 bis 6 mm, einen Außendurchmesser von 4 bis 8 mm und eine Wanddicke von 1 bis 2 mm. Geeignet sind vor allem auch Ringe der Geometrie 7 mm x 3 mm x 4 mm (Außendurchmesser x Länge x Innendurchmesser) als Trägerkörper. Die Schichtdicke der Schale (b) aus einer Molybdän und mindestens ein weiteres Metall enthaltenden Multimetalloxidmasse liegt in der Regel bei 5 bis 1000 $\mu$m. Bevorzugt sind 10 bis 800 $\mu$m, besonders bevorzugt 50 bis 600 $\mu$m und ganz besonders bevorzugt 80 bis 500 $\mu$m.

Beispiele für Mo-Bi-Fe-O-haltige Multimetalloxide sind Mo-Bi-Fe-Cr-O- oder Mo-Bi-Fe-Zr-O-haltige Multimetalloxide. Bevorzugte Systeme sind beispielsweise beschrieben in US 4,547,615 ($Mo_{12}BiFe_{0,1}Ni_8ZrCr_3K_{0,2}O_x$ und $Mo_{12}BiFe_{0,1}Ni_8AlCr_3K_{0,2}O_x$), US 4,424,141 ($Mo_{12}BiFe_3Co_{4,5}Ni_{2,5}P_{0,5}K_{0,1}O_x$ + $SiO_2$), DE-A 25 30 959 ($Mo_{12}BiFe_3Co_{4,5}Ni_{2,5}Cr_{0,5}K_{0,1}O_x$, $Mo_{13,75}BiFe_3Co_{4,5}Ni_{2,5}Ge_{0,5}K_{0,8}O_x$, $Mo_{12}BiFe_3Co_{4,5}Ni_{2,5}Mn_{0,5}K_{0,1}O_x$ und $Mo_{12}BiFe_3Co_{4,5}Ni_{2,5}La_{0,5}K_{0,1}O_x$), US 3,911,039 ($Mo_{12}BiFe_3Co_{4,5}Ni_{2,5}Sn_{0,5}K_{0,1}O_x$), DE-A 25 30 959 und DE-A 24 47 825 ($Mo_{12}BiFe_3Co_{4,5}Ni_{2,5}W_{0,5}K_{0,1}O_x$).

Geeignete Multimetalloxide und deren Herstellung sind weiterhin beschrieben in US 4,423,281 ($Mo_{12}BiNi_8Pb_{0,5}Cr_3K_{0,2}O_x$ und $Mo_{12}Bi_bNi_7Al_3Cr_{0,5}K_{0,5}O_x$), US 4,336,409 ($Mo_{12}BiNi_6Cd_2Cr_3P_{0,5}O_x$), DE-A 26 00 128 ($Mo_{12}BiNi_{0,5}Cr_3P_{0,5}Mg_{7,5}K_{0,1}O_x$ + $SiO_2$) und DE-A 24 40 329 ($Mo_{12}BiCo_{4,5}Ni_{2,5}Cr_3P_{0,5}K_{0,1}O_x$).

Besonders bevorzugte katalytisch aktive, Molybdän und mindestens ein weiteres Metall enthaltende Multimetalloxide weisen die allgemeine Formel (Ia) auf:

$$Mo_{12}Bi_aFe_bCO_cNi_dCr_eX^1_fX^2_gO_y \qquad (Ia),$$

mit

$X^1$ = Si, Mn und/oder Al,

$X^2$ = Li, Na, K, Cs und/oder Rb,

$$0,2 \leq a \leq 1,$$

$$0,5 \leq b \leq 10,$$

$$0 \leq c \leq 10,$$

$$0 \leq d \leq 10,$$

$$2 \leq c + d \leq 10$$

$$0 \leq e \leq 2,$$

$$0 \leq f \leq 10$$

$$0 \leq g \leq 0,5$$

y = eine Zahl, die unter der Voraussetzung der Ladungsneutralität durch die Wertigkeit und Häufigkeit der von Sauerstoff verschiedenen Elemente in (Ia) bestimmt wird.

Bevorzugt sind Katalysatoren, deren katalytisch aktive Oxidmasse von den beiden Metallen Co und Ni nur Co aufweist (d = 0). Bevorzugte ist $X^1$ Si und/oder Mn und $X^2$ ist vorzugsweise K, Na und/oder Cs, besonders bevorzugt ist $X^2$ = K.

Der stöchiometrische Koeffizient a in Formel (Ia) beträgt vorzugsweise $0,4 \leq a \leq 1$, besonders bevorzugt $0,4 \leq a \leq 0,95$. Der Wert für die Variable b liegt vorzugsweise im Bereich $1 \leq b \leq 5$ und besonders bevorzugt im Bereich $2 \leq b \leq 4$. Die Summe der stöchiometrischen Koeffizienten c + d liegt bevorzugt im Bereich $4 \leq c + d \leq 8$, und besonders bevorzugt im Bereich $6 \leq c + d \leq 8$. Der stöchiometrische Koeffizient e liegt bevorzugt im Bereich $0,1 \leq e \leq 2$, und besonders bevorzugt im Bereich $0,2 \leq e \leq 1$. Der stöchiometrische Koeffizient g ist zweckmäßigerweise $\geq 0$. Bevorzugt ist $0,01 \leq g \leq 0,5$ und besonders bevorzugt gilt $0,05 \leq g \leq 0,2$.

Der Wert für den stöchiometrischen Koeffizienten des Sauerstoffs, y, ergibt sich aus der Wertigkeit und Häufigkeit der Kationen unter der Voraussetzung der Ladungsneutralität. Günstig sind Schalenkatalysatoren mit katalytisch aktiven Oxidmassen, deren molares Verhältnis von Co/Ni wenigstens 2:1, bevorzugt wenigstens 3:1 und besonders bevorzugt wenigstens 4:1 beträgt. Am besten liegt nur Co vor.

Der Schalenkatalysator wird hergestellt, indem man auf den Trägerkörper mittels eines Bindemittels eine Schicht enthaltend das Molybdän und mindestens ein weiteres Metall enthaltende Multimetalloxid aufbringt, den beschichteten Trägerkörper trocknet und kalziniert.

Erfindungsgemäß zu verwendende feinteilige, Molybdän und mindestens ein weiteres Metall enthaltende Multimetalloxide sind grundsätzlich dadurch erhältlich, dass man von Ausgangsverbindungen der elementaren Konstituenten der katalytisch aktiven Oxidmasse ein inniges Trockengemisch erzeugt und das innige Trockengemisch bei einer Temperatur von 150 bis 650 °C thermisch behandelt.

Herstellung des Multimetalloxid-Katalysators

Zur Herstellung von solchen und anderen geeigneten feinteiligen Multimetalloxidmassen geht man von bekannten Ausgangsverbindungen der von Sauerstoff verschiedenen elementaren Konstituenten der gewünschten Multimetalloxidmasse im jeweiligen stöchiometrischen Verhältnis aus, und erzeugt aus diesen ein möglichst inniges, vorzugsweise feinteiliges Trockengemisch, welches dann der thermischen Behandlung unterworfen wird. Dabei kann es sich bei den Quellen entweder bereits um Oxide handeln, oder um solche Verbindungen, die durch Erhitzen, wenigstens in Anwesenheit von Sauerstoff, in Oxide überführbar sind. Neben den Oxiden kommen daher als Ausgangsverbindungen vor allem Halogenide, Nitrate, Formiate, Oxalate, Acetate, Carbonate oder Hydroxide in Betracht.

Geeignete Ausgangsverbindungen des Molybdäns sind auch dessen Oxoverbindungen (Molybdate) oder die von diesen abgeleiteten Säuren.

Geeignete Ausgangsverbindungen von Bi, Cr, Fe und Co sind insbesondere deren Nitrate.

Das innige Vermischen der Ausgangsverbindungen kann prinzipiell in trockener Form oder in Form der wässrigen Lösungen oder Suspensionen erfolgen.

Eine wässrige Suspension kann beispielsweise durch das Vereinigen einer Lösung, die wenigstens Molybdän enthält, und einer wässrigen Lösung, die die übrigen Metalle enthält, hergestellt werden. Alkalimetalle oder Erdalkalimetalle können in beiden Lösungen vorliegen. Durch Vereinigen der Lösungen wird eine Fällung durchgeführt, die zur Bildung einer Suspension führt. Die Temperatur der Fällung kann höher als Raumtemperatur, bevorzugt von 30 °C bis 95 °C, und besonders bevorzugt von 35 °C bis 80 °C, sein. Die Suspension kann danach über einen gewissen Zeitraum bei erhöhter Temperatur gealtert werden. Der Alterungszeitraum liegt im Allgemeinen zwischen 0 und 24 Stunden, bevorzugt zwischen 0 und 12 Stunden, und besonders bevorzugt zwischen 0 und 8 Stunden. Die Temperatur der Alterung ist im Allgemeinen zwischen 20 °C und 99 °C, bevorzugt zwischen 30 °C und 90 °C, und besonders bevorzugt zwischen 35 °C und 80 °C. Während der Fällung und Alterung der Suspension wird diese im Allgemeinen durch Rühren gemischt. Der pH-Wert der gemischten Lösungen oder Suspension liegt im Allgemeinen zwischen pH 1 und pH 12, bevorzugt zwischen pH 2 und pH 11 und besonders bevorzugt zwischen pH 3 und pH 10.

Durch Entfernen des Wassers wird ein Feststoff hergestellt, der eine innige Mischung der zugegebenen Metallkomponenten darstellt. Der Trocknungsschritt kann im Allgemeinen durch Eindampfen, Sprühtrocknen oder Gefriertrocknen oder dergleichen durchgeführt werden. Bevorzugt erfolgt die Trocknung durch Sprühtrocknen. Die Suspension wird hierzu bei erhöhter Temperatur mit einem Sprühkopf, dessen Temperatur sich im Allgemeinen bei 120 °C bis 300 °C befindet, zerstäubt und das getrocknete Produkt bei einer Temperatur von >60 °C gesammelt. Die Restfeuchte, bestimmt durch Trocknung des Sprühpulvers bei 120 °C, beträgt im Allgemeinen weniger als 20 Gew.-%, bevorzugt weniger als 15 Gew. % und besonders bevorzugt weniger als 12 Gew. %.

Zur Herstellung von Vollmaterialkatalysatoren wird das Sprühpulver in einem weiteren Schritt in einen Formkörper überführt. Als Formungshilfsmittel (Gleitmittel) kommen z.B. Wasser, Bortrifluorid oder Graphit in Betracht. Bezogen auf die zum Katalysatorvorläuferformkörper zu formende Masse werden in der Regel ≤ 10 Gew.-%, meist ≤ 6 Gew.-%, vielfach ≤ 4 Gew.- % an Formungshilfsmittel zugesetzt. Üblicherweise beträgt die vorgenannte Zusatzmenge >0,5 Gew.-%. Bevorzugtes Gleithilfsmittel ist Graphit.

Die thermische Behandlung des Katalysatorvorläuferformkörpers erfolgt in der Regel bei Temperaturen, die 350 °C überschreiten. Normalerweise wird im Rahmen der thermischen Behandlung die Temperatur von 650 °C jedoch nicht überschritten. Erfindungsgemäß vorteilhaft wird im Rahmen der thermischen Behandlung die Temperatur von 600 °C, bevorzugt die Temperatur von 550 °C und besonders bevorzugt die Temperatur von 500 °C nicht überschritten. Ferner wird beim erfindungsgemäßen Verfahren im Rahmen der thermischen Behandlung des Katalysatorvorläuferformkörpers vorzugsweise die Temperatur von 380 °C, mit Vorteil die Temperatur von 400 °C, mit besonderem Vorteil die Temperatur von 420 °C und ganz besonders bevorzugt die Temperatur von 440 °C überschritten. Dabei kann die thermische Behandlung in ihrem zeitlichen Ablauf auch in mehrere Abschnitte gegliedert sein. Beispielsweise kann zunächst eine thermische Behandlung bei einer Temperatur von 150 bis 350 °C, vorzugsweise von 220 bis 280 °C, und daran anschließend eine thermische Behandlung bei einer Temperatur von 400 bis 600 °C, vorzugsweise von 430 bis 550 °C durchgeführt werden. Normalerweise nimmt die thermische Behandlung des Katalysatorvorläuferformkörpers mehrere Stunden (meist mehr als 5 h) in Anspruch. Häufig erstreckt sich die Gesamtdauer der thermischen Behandlung auf mehr als 10 h. Meist werden im Rahmen der thermischen Behandlung des Katalysatorvorläuferformkörpers Behandlungsdauern von 45 h bzw. 35 h nicht überschritten. Oft liegt die Gesamtbehandlungsdauer unterhalb von 30 h. Vorzugsweise werden bei der thermischen Behandlung des Katalysatorvorläuferformkörpers 500 °C nicht überschritten und die Behandlungsdauer im Temperaturfenster von ≥400 °C erstreckt sich auf 5 bis 30 h.

Die thermische Behandlung (Kalzination) der Katalysatorvorläuferformkörper kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemisch aus Inertgas, $NH_3$, CO und/oder $H_2$ oder Methan) erfolgen. Selbstredend kann die thermische Behandlung auch unter Vakuum ausgeführt werden. Prinzipiell kann die thermische Behandlung der Katalysatorvorläuferformkörper in den unterschiedlichsten Ofentypen wie z.B. beheizbare Umluftkammern, Hordenöfen, Drehrohröfen, Bandkalzinierer oder Schachtöfen durchgeführt werden. Bevorzugt erfolgt die thermische Behandlung der Katalysatorvorläuferformkörper in einer Bandkalziniervorrichtung, wie sie die DE-A 10046957 und die WO 02/24620 empfehlen. Die thermische Behandlung der Katalysatorvorläuferformkörper unterhalb von 350 °C verfolgt in der Regel die thermische Zersetzung der in den Katalysatorvorläuferformkörpern enthaltenen Quellen der elementaren Konstituenten des angestrebten Katalysators. Häufig erfolgt beim erfindungsgemäßen Verfahren diese Zersetzungsphase im Rahmen des Aufheizens auf Temperaturen < 350 °C.

Die katalytisch aktive Multimetalloxidmasse kann Chromoxid enthalten. Als Ausgangsstoffe kommen neben den Oxiden vor allem Halogenide, Nitrate, Formiate, Oxalate, Acetate, Carbonate und/oder Hydroxide in Betracht. Die thermische Zersetzung von Chrom(III)verbindungen zu Chrom(III)oxid erfolgt unabhängig von der An- oder Abwesenheit von Sauerstoff, hauptsächlich zwischen 70-430 °C über mehrere Chrom(VI)-haltige Zwischenstufen (siehe zum Beispiel J. Therm. Anal. Cal., 72, 2003, 135 und Env. Sei. Tech. 47, 2013, 5858). Die Anwesenheit von Chrom(VI)oxid ist für die katalytische Oxydehydrierung von Alkenen zu Dienen, speziell von Butenen zu Butadien, nicht erforderlich. Auf Grund der Toxizität und Umweltschädlichkeit von Cr(VI)oxid soll die Aktivmasse daher weitgehend frei von Chrom(VI)oxid sein. Der Chrom(VI)oxid-Gehalt hängt weitgehend von Kalzinationsbedingungen ab, insbesondere der höchsten Temperatur im Kalzinierschritt ab und von dessen Haltedauer. Hier gilt, dass je höher die Temperatur ist und je länger die Haltedauer, desto geringer ist der Gehalt an Chrom(VI)oxid.

Der nach der Kalzination erhaltene Formkörper aus katalytisch aktiver Multimetalloxidmasse kann als Vollmaterialkatalysator eingesetzt werden. Zur Herstellung eines Schalenkatalysators kann der Formkörper anschließend durch Mahlen in ein feinteiliges Pulver überführt werden, das dann mit Hilfe eines flüssigen Bindemittels auf die äußere Oberfläche des Trägerkörpers aufgebracht wird. Die Feinheit der auf die Oberfläche des Trägerkörpers aufzubringenden katalytisch aktiven Oxidmasse wird dabei an die gewünschte Schalendicke angepasst.

Zur Herstellung von Schalenkatalysatoren geeignete Trägermaterialien sind poröse oder bevorzugt unporöse Aluminiumoxide, Siliciumdioxid, Zirkondioxid, Siliciumcarbid oder Silikate wie Magnesium- oder Aluminiumsilicat (z.B. Steatit des Typs C 220 der Fa. CeramTec). Die Materialien der Trägerkörper sind chemisch inert.

Die Trägermaterialien können porös oder nicht porös sein. Vorzugsweise ist das Trägermaterial nicht porös (Gesamtvolumen der Poren, bezogen auf das Volumen des Trägerkörpers, vorzugsweise ≤ 1 Vol-%).

Bevorzugte Hohlzylinder als Trägerkörper weisen eine Länge von 2 bis 10 mm und einen Außendurchmesser von 4 bis 10 mm auf. Die Wanddicke liegt darüber hinaus vorzugsweise bei 1 bis 4 mm. Besonders bevorzugte ringförmige Trägerkörper besitzen eine Länge von 2 bis 6 mm, einen Außendurchmesser von 4 bis 8 mm und eine Wanddicke von 1 bis 2 mm. Ein Beispiel sind Ringe der Geometrie 7 mm x 3 mm x 4 mm (Außendurchmesser x Länge x Innendurchmesser) als Trägerkörper.

Die Schichtdicke D aus einer Molybdän und mindestens ein weiteres Metall enthaltenden Multimetalloxidmasse liegt in der Regel bei 5 bis 1000 $\mu$m. Bevorzugt sind 10 bis 800 $\mu$m, besonders bevorzugt 50 bis 600 $\mu$m und ganz besonders bevorzugt 80 bis 500 $\mu$m.

Das Aufbringen des Molybdän und mindestens ein weiteres Metall enthaltenden Multimetalloxids auf die Oberfläche des Trägerkörpers kann entsprechend den im Stand der Technik beschriebenen Verfahren erfolgen, beispielsweise wie in US-A 2006/0205978 sowie EP-A 0 714 700 beschrieben.

Im Allgemeinen werden die feinteiligen Massen auf die Oberfläche des Trägerkörpers bzw. auf die Oberfläche der ersten Schicht mit Hilfe eines flüssigen Bindemittels aufgebracht. Als flüssiges Bindemittel kommt z.B. Wasser, ein organisches Lösungsmittel oder eine Lösung einer organischen Substanz (z.B. eines organischen Lösungsmittels) in Wasser oder in einem organischen Lösungsmittel in Betracht.

Besonders vorteilhaft wird als flüssiges Bindemittel eine Lösung bestehend aus 20 bis 95 Gew.-% Wasser und 5 bis 80 Gew.-% einer organischen Verbindung verwendet. Vorzugsweise beträgt der organische Anteil an den vorgenannten flüssigen Bindemitteln 10 bis 50 Gew.-% und besonders bevorzugt 10 bis 30 Gew.-%.

Bevorzugt sind generell solche organischen Bindemittel bzw. Bindemittelanteile, deren Siedepunkt oder Sublimationstemperatur bei Normaldruck (1 atm) ≥ 100 °C, vorzugsweise ≥ 150 °C beträgt. Ganz besonders bevorzugt liegt der Siedepunkt oder Sublimationspunkt solcher organischen Bindemittel bzw. Bindemittelanteile bei Normaldruck gleichzeitig unterhalb der im Rahmen der Herstellung des Molybdän enthaltenden feinteiligen Multimetalloxids angewandten höchsten Kalzinationstemperatur. Üblicherweise liegt diese höchste Kalzinationsstemperatur bei ≤ 600 °C, häufig bei ≤ 500 °C.

Beispielhaft genannt seien als organische Bindemittel ein- oder mehrwertige organische Alkohole wie z.B. Ethylenglykol, 1,4-Butandiol, 1,6-Hexandiol oder Glycerin, ein- oder mehrwertige organische Carbonsäuren wie Propionsäure, Oxalsäure, Malonsäure, Glutarsäure oder Maleinsäure, Aminoalkohole wie Ethanolamin oder Diethanolamin sowie ein- oder mehrwertige organische Amide wie Formamid. Als in Wasser, in einer organischen Flüssigkeit oder in einem Gemisch aus Wasser und einer organischen Flüssigkeit lösliche organische Bindemittelpromotoren sind z.B. Monosaccharide und Oligosaccharide wie Glucose, Fructose, Saccharose und/oder Lactose geeignet.

Besonders bevorzugte flüssige Bindemittel sind Lösungen, die aus 20 bis 95 Gew.-% Wasser und 5 bis 80 Gew.-% Glycerin bestehen. Vorzugsweise beträgt der Glycerinanteil in diesen wässrigen Lösungen 5 bis 50 Gew.-% und besonders bevorzugt 8 bis 35 Gew.-%.

Das Aufbringen des Molybdän enthaltenden feinteiligen Multimetalloxids kann in der Weise erfolgen, dass man die feinteilige Masse aus Molybdän enthaltendem Multimetalloxid in dem flüssigen Bindemittel dispers verteilt und die dabei resultierende Suspension auf bewegte und gegebenenfalls heiße Trägerkörper aufsprüht, wie beschrieben in DE-A 1642921, DE-A 2106796 und die DE-A 2626887. Nach Beendigung des Aufsprühens kann, wie in DE-A 2909670 beschrieben, durch Überleiten von heißer Luft der Feuchtigkeitsgehalt der resultierenden Schalenkatalysatoren verringert werden.

Zu dem feinteiligen Multimetalloxid, welches auf den Träger aufgebracht wird, können zusätzlich Porenbildner wie Malonsäure, Melamin, Nonylphenolethoxylat, Stearinsäure, Glucose, Stärke, Fumarsäure und Bernsteinsäure zugesetzt werden, um eine geeignete Porenstruktur des Katalysators zu erzeugen und die Stofftransporteigenschaften zu verbessern. Der erfindungsgemäß verwendete Katalysator enthält vorzugsweise keine Porenbildner.

Bevorzugt wird man aber die Trägerkörper zunächst mit dem flüssigen Bindemittel befeuchten und nachfolgend die feinteilige Masse aus Multimetalloxid dadurch auf die Oberfläche des mit Bindemittel angefeuchteten Trägerkörpers aufbringen, dass man die befeuchteten Trägerkörper in der feinteiligen Masse wälzt. Zur Erzielung der gewünschten Schichtdicke wird das vorstehend beschriebene Verfahren vorzugsweise mehrmals wiederholt, d. h. der grundbeschichtete Trägerkörper wird wiederum befeuchtet und dann durch Kontakt mit trockener feinteiliger Masse beschichtet.

Für eine Durchführung des Verfahrens im technischen Maßstab empfiehlt sich die Anwendung des in der DE-A 2909671 offenbarten Verfahrens, jedoch vorzugsweise unter Verwendung der in der EP-A 714700 empfohlenen Bindemittel. D.h., die zu beschichtenden Trägerkörper werden in einen vorzugsweise geneigten (der Neigungswinkel beträgt in der Regel 30 bis 90°) rotierenden Drehbehälter (z.B. Drehteller oder Dragierkessel) gefüllt.

Die Temperaturen, die notwendig sind, um das Entfernen des Haftvermittlers zu bewirken, liegen unterhalb der höchsten Kalzinationstemperatur des Katalysators, im Allgemeinen zwischen 200 °C und 600 °C. Bevorzugt wird der Katalysator auf 240 °C bis 500 °C erhitzt, und besonders bevorzugt auf Temperaturen zwischen 260 °C und 400 °C. Die Zeit zum Entfernen des Haftvermittlers kann mehrere Stunden betragen. Der Katalysator wird im Allgemeinen zwischen 0.5 und 24 Stunden auf die genannte Temperatur erhitzt, um den Haftvermittler zu entfernen. Bevorzugt ist die Zeit zwischen 1.5 und 8 Stunden und besonders bevorzugt zwischen 2 und 6 Stunden. Eine Umströmung des Katalysators mit einem Gas kann das Entfernen des Haftvermittlers beschleunigen. Das Gas ist bevorzugt Luft oder Stickstoff und besonders bevorzugt Luft. Das Entfernen des Haftvermittlers kann zum Beispiel in einem gasdurchströmten Ofen oder in einer geeigneten Trocknungsapparatur, beispielsweise einem Bandtrockner, erfolgen.

Oxidative Dehydrierung (Oxidehydrierung, ODH)

In mehreren Produktionszyklen (i) wird eine oxidative Dehydrierung von n-Butenen zu Butadien durchgeführt, indem ein n-Butene enthaltendes Ausgangsgasgemisch mit einem Sauerstoff enthaltenden Gas und gegebenenfalls zusätzlichem Inertgas oder Wasserdampf gemischt und in einem Festbettreaktor bei einer Temperatur von 350 bis 490 °C mit einem in einem Katalysatorfestbett angeordneten Katalysator in Kontakt gebracht wird. Die genannten Temperaturen beziehen sich auf die Temperatur des Wärmeaustauschmittels.

Die Reaktionstemperatur der Oxidehydrierung wird im Allgemeinen durch ein Wärmeaustauschmittel, welches sich um die Reaktionsrohre herum befindet, kontrolliert. Als solche flüssige Wärmeaustauschmittel kommen z. B. Schmelzen von Salzen wie Kaliumnitrat, Kaliumnitrit, Natriumnitrit und/oder Natriumnitrat sowie Schmelzen von Metallen wie Natrium, Quecksilber und Legierungen verschiedener Metalle in Betracht. Aber auch ionische Flüssigkeiten oder Wärmeträgeröle sind einsetzbar. Die Temperatur des Wärmeaustauschmittels beträgt von 350 bis 490 °C, bevorzugt von 360 bis 450 °C und besonders bevorzugt von 365 bis 420 °C.

Auf Grund der Exothermie der ablaufenden Reaktionen kann die Temperatur in bestimmten Abschnitten des Reaktorinneren während der Reaktion höher liegen als diejenige des Wärmeaustauschmittels, und es bildet sich ein so genannter Hotspot aus. Die Lage und Höhe des Hotspots ist durch die Reaktionsbedingungen festgelegt, aber sie kann auch durch das Verdünnungsverhältnis der Katalysatorschicht oder den Durchfluss an Mischgas reguliert werden.

Die Oxidehydrierung kann in allen aus dem Stand der Technik bekannten Festbettreaktoren durchgeführt werden, wie beispielsweise im Hordenofen, im Festbettrohr- oder Festbettrohrbündelreaktor oder im Plattenwärmetauscherreaktor. Ein Rohrbündelreaktor ist bevorzugt.

Weiterhin kann die Katalysatorschicht, die im Reaktor eingerichtet ist, wie oben beschrieben aus einer einzelnen Schicht oder aus 2 oder mehr Schichten bestehen. Diese Schichten können aus reinem Katalysator bestehen oder mit einem Material verdünnt sein, das nicht mit dem Ausgangsgas oder Komponenten aus dem Produktgas der Reaktion reagiert. Weiterhin können die Katalysatorschichten aus Vollmaterial oder geträgerten Schalenkatalysatoren bestehen.

Als Ausgangsgas können reine n-Butene (1-Buten und/oder cis-/trans-2-Buten), aber auch ein Butene enthaltendes Gasgemisch eingesetzt werden. Ein solches kann beispielsweise durch nicht-oxidative Dehydrierung von n-Butan erhalten werden. Es kann auch eine Fraktion verwendet werden, die als Hauptbestandteil n-Butene (1-Buten und/oder 2-Buten) enthält, und aus der $C_4$-Fraktion des Naphtha-Crackens durch Abtrennen von Butadien und Isobuten erhalten wurde. Des Weiteren können auch Gasgemische als Ausgangsgas eingesetzt werden, die reines 1-Buten, cis-2-Buten, trans-2-Buten oder Mischungen daraus umfassen, und durch Dimerisierung von Ethylen erhalten wurden. Ferner können als Ausgangsgas n-Butene enthaltende Gasgemische eingesetzt werden, die durch katalytisches Wirbelschichtcracken (Fluid Catalytic Cracking, FCC) erhalten wurden.

In einer Ausführungsform des erfindungsgemäßen Verfahrens wird das n-Butene enthaltende Ausgangsgasgemisch durch nicht-oxidative Dehydrierung von n-Butan erhalten. Durch die Kopplung einer nicht-oxidativen katalytischen Dehydrierung mit der oxidativen Dehydrierung der gebildeten n-Butene kann eine hohe Ausbeute an Butadien, bezogen auf eingesetztes n-Butan, erhalten werden. Bei der nicht-oxidativen katalytischen n-Butan-Dehydrierung wird ein Gasgemisch erhalten, das neben Butadien 1-Buten, 2-Buten und nicht umgesetztem n-Butan Nebenbestandteile enthält. Übliche Nebenbestandteile sind Wasserstoff, Wasserdampf, Stickstoff, CO und $CO_2$, Methan, Ethan, Ethen, Propan und Propen. Die Zusammensetzung des die erste Dehydrierzone verlassenden Gasgemischs kann abhängig von der Fahrweise der Dehydrierung stark variieren. So weist bei Durchführung der Dehydrierung unter Einspeisung von Sauerstoff und zusätzlichem Wasserstoff das Produktgasgemisch einen vergleichsweise hohen Gehalt an Wasserdampf und Kohlenstoffoxiden auf. Bei Fahrweisen ohne Einspeisung von Sauerstoff weist das Produktgasgemisch der nicht-oxidativen Dehydrierung einen vergleichsweise hohen Gehalt an Wasserstoff auf.

Der Produktgasstrom der nicht-oxidativen n-Butan-Dehydrierung enthält typischerweise 0,1 bis 15 Vol.-% Butadien, 1 bis 15 Vol.-% 1-Buten, 1 bis 25 Vol.-% 2-Buten (cis/trans-2-Buten), 20 bis 70 Vol.-% n-Butan, 1 bis 70 Vol.-% Wasserdampf, 0 bis 10 Vol.-% leichtsiedende Kohlenwasserstoffe (Methan, Ethan, Ethen, Propan und Propen), 0,1 bis 40 Vol.-% Wasserstoff, 0 bis 70 Vol.-% Stickstoff und 0 bis 5 Vol.-% Kohlenstoffoxide. Der Produktgasstrom der nicht-oxidativen Dehydrierung kann ohne weitere Aufarbeitung der oxidativen Dehydrierung zugeführt werden.

Weiterhin können in dem Ausgangsgas der Oxidehydrierung beliebige Verunreinigungen in einem Bereich, in dem die Wirkung der vorliegenden Erfindung nicht gehemmt wird, vorhanden sein. Bei der Herstellung von Butadien aus n-Butenen (1-Buten und cis-/trans-2-Buten) können als Verunreinigungen gesättigte und ungesättigte, verzweigte und unverzweigte Kohlenwasserstoffe, wie z. B. Methan, Ethan, Ethen, Acetylen, Propan, Propen, Propin, n-Butan, iso-Butan, iso-Buten, n-Pentan sowie Diene wie 1,2-Butadien genannt werden. Die Mengen an Verunreinigungen betragen im Allgemeinen 70 % oder weniger, vorzugsweise 30 % oder weniger, weiter bevorzugt 10 % oder weniger und besonders bevorzugt 1 % oder weniger. Die Konzentration an linearen Monoolefinen mit 4 oder mehr Kohlenstoffatomen (n-Butene und höherer Homologe) im Ausgangsgas ist nicht besonders eingeschränkt; sie beträgt im Allgemeinen 35,0-99,99 Vol.-%, vorzugsweise 71,0-99,0 Vol.-% und noch mehr bevorzugt 75,0-95,0 Vol.-%.

Zur Durchführung der oxidativen Dehydrierung bei Vollumsatz von Butenen wird ein Gasgemisch benötigt, welches ein molares Sauerstoff: n-Butene-Verhältnis von mindestens 0,5 aufweist. Bevorzugt wird bei einem Sauerstoff: n-Butene-Verhältnis von 0,55 bis 10 gearbeitet. Zur Einstellung dieses Wertes kann das Ausgangsstoffgas mit Sauerstoff oder einem sauerstoffhaltigem Gas, beispielsweise Luft, und gegebenenfalls zusätzlichem Inertgas oder Wasserdampf vermischt werden. Das erhaltene sauerstoffhaltige Gasgemisch wird dann der Oxidehydrierung zugeführt.

Das molekularen Sauerstoff enthaltende Gas ist ein Gas, das im Allgemeinen mehr als 10 Vol.-%, vorzugsweise mehr als 15 Vol.-% und noch mehr bevorzugt mehr als 20 Vol.-% molekularen Sauerstoff umfasst, und konkret ist dies vorzugsweise Luft. Die Obergrenze für den Gehalt an molekularem Sauerstoff beträgt im Allgemeinen 50 Vol.-% oder weniger, vorzugsweise 30 Vol.-% oder weniger und noch mehr bevorzugt 25 Vol.-% oder weniger. Darüber hinaus können in dem molekularen Sauerstoff enthaltenden Gas beliebige Inertgase in einem Bereich, in dem die Wirkung der vorliegenden Erfindung nicht gehemmt wird, vorhanden sein. Als mögliche Inertgase können Stickstoff, Argon, Neon, Helium, CO, $CO_2$ und Wasser genannt werden. Die Menge an Inertgase beträgt für Stickstoff im Allgemeinen 90 Vol.-% oder weniger, vorzugsweise 85 Vol.-% oder weniger und noch mehr bevorzugt 80 Vol.-% oder weniger. Im Falle anderer Bestandteile als Stickstoff beträgt sie im Allgemeinen 10 Vol.-% oder weniger, vorzugsweise 1 Vol.-% oder weniger. Wird diese Menge zu groß, wird es immer schwieriger, die Reaktion mit dem erforderlichen Sauerstoff zu versorgen.

Ferner können zusammen mit dem Mischgas aus Ausgangsgas und dem molekularen Sauerstoff enthaltenden Gas auch inerte Gase wie Stickstoff und weiterhin Wasser (als Wasserdampf) enthalten sein. Stickstoff ist zur Einstellung der Sauerstoffkonzentration und zur Verhinderung der Ausbildung eines explosionsfähigen Gasgemischs vorhanden, das gleiche gilt für Wasserdampf. Wasserdampf ist ferner zur Kontrolle des Verkokens des Katalysators und zur Abfuhr der Reaktionswärme vorhanden. Vorzugsweise werden Wasser (als Wasserdampf) und Stickstoff in das Mischgas eingemischt und in den Reaktor eingeleitet. Beim Einleiten von Wasserdampf in den Reaktor wird vorzugsweise ein Anteil von 0,2-5,0 (Volumenteile) vorzugsweise 0,5-4 und noch mehr bevorzugt 0,8-2,5, bezogen auf die Einleitungsmenge an vorgenanntem Ausgangsgas, eingeleitet. Beim Einleiten von Stickstoffgas in den Reaktor wird vorzugsweise ein Anteil von 0,1-8,0 (Volumenteile), vorzugsweise mit 0,5-5,0 und noch mehr bevorzugt 0,8-3,0 bezogen auf die Einleitungsmenge an vorgenanntem Ausgangsgas eingeleitet.

Der Anteil des die Kohlenwasserstoffe enthaltenden Ausgangsgases im Mischgas beträgt im Allgemeinen 4,0 Vol.-% oder mehr, vorzugsweise 6,0 Vol.-% oder mehr und noch mehr bevorzugt 8,0 Vol.-% oder mehr. Andererseits liegt die Obergrenze bei 20 Vol.-% oder weniger, vorzugsweise bei 16,0 Vol.-% oder weniger und noch mehr bevorzugt bei 13,0 Vol.-% oder weniger. Um die Bildung von explosiven Gasgemischen sicher zu vermeiden, wird vor dem Erhalt des Mischgases zunächst Stickstoffgas in das Ausgangsgas oder in das molekularen Sauerstoff enthaltende Gas eingeleitet, das Ausgangsgas und das molekularen Sauerstoff enthaltende Gas wird gemischt und so das Mischgas erhalten, und dieses Mischgas wird nun vorzugsweise eingeleitet.

Während des stabilen Betriebs ist die Verweildauer im Reaktor in der vorliegenden Erfindung nicht besonders eingeschränkt, aber die Untergrenze beträgt im Allgemeinen 0,3 s oder mehr, vorzugsweise 0,7 s oder mehr und noch mehr bevorzugt 1,0 s oder mehr. Die Obergrenze beträgt 5,0 s oder weniger, vorzugsweise 3,5 s oder weniger und noch mehr bevorzugt 2,5 s oder weniger. Das Verhältnis von Durchfluss an Mischgas bezogen auf die Katalysatormenge im Reaktorinnern beträgt 500-8000 $h^{-1}$, vorzugsweise 800-4000 $h^{-1}$ und noch mehr bevorzugt 1200-3500 $h^{-1}$. Die Last des Katalysators an Butenen (ausgedrückt in $g_{Butene}/(g_{Katalysator}$*Stunde) beträgt im Allgemeinen im stabilen Betrieb 0,1-5,0 $h^{-1}$, vorzugsweise 0,2-3,0 $h^{-1}$ und noch mehr bevorzugt 0,25-1,0 $h^{-1}$. Volumen und Masse des Katalysators beziehen sich auf den kompletten Katalysator bestehend aus Träger und Aktivmasse.

Regenerierung des Multimetalloxid-Katalysators

Erfindungsgemäß wird ein Regenerierschritt (ii) zwischen jeweils zwei Produktionsschritten (i) durchgeführt. Der Regenerierschritt (ii) wird im Allgemeinen durchgeführt, bevor der Umsatzverlust bei konstanter Temperatur größer 25% beträgt. Der Regenerierschritt (ii) wird durch Überleiten eines sauerstoffhaltigen Regeneriergasgemischs bei einer Temperatur von 350 bis 490 °C über das Katalysatorfestbett, wodurch der auf dem Multimetalloxid-Katalysator abgeschiedenen Kohlenstoffs abgebrannt wird, durchgeführt. Vorzugsweise werden pro Regenerierzyklus (ii) 5 bis 50 Gew.-% des auf dem Katalysator abgeschiedenen Kohlenstoffs abgebrannt.

Das im Regenerierschritt (ii) eingesetzte sauerstoffhaltige Regeneriergasgemisch enthält im Allgemeinen ein sauerstoffhaltiges Gas und zusätzliche Inertgase, Wasserdampf und/oder Kohlenwasserstoffe. Im Allgemeinen enthält es 0,5 bis 22 Vol.-%, vorzugsweise 1 bis 20 Vol.-% und insbesondere 2 bis 18 Vol.-% Sauerstoff.

Ein bevorzugtes sauerstoffhaltiges Gas, welches in dem Regeneriergasgemisch vorliegt, ist Luft. Zur Erzeugung des sauerstoffhaltigen Regeneriergasgemischs können dem sauerstoffhaltigen Gas gegebenenfalls noch zusätzlich Inertgase, Wasserdampf und/oder Kohlenwasserstoffe zugemischt werden. Als mögliche Inertgase können Stickstoff, Argon, Neon, Helium, CO und $CO_2$ genannt werden. Die Menge an Inertgasen beträgt für Stickstoff im Allgemeinen 90 Vol.-% oder weniger, vorzugsweise 85 Vol.-% oder weniger und noch mehr bevorzugt 80 Vol.-% oder weniger. Im Falle anderer Bestandteile als Stickstoff beträgt sie im Allgemeinen 10 Vol.-% oder weniger, vorzugsweise 1 Vol.-% oder weniger. Die Menge an Sauerstoff enthaltendem Gas wird so gewählt, dass der Volumenanteil an molekularem Sauerstoff im Regeneriergasgemisch zu Beginn der Regeneration 0-50 %, vorzugsweise 0,5-22 % und noch mehr bevorzugt 1-10 % beträgt. Der Anteil an molekularem Sauerstoff kann im Laufe der Regeneration erhöht werden.

Ferner kann in dem sauerstoffhaltigen Regeneriergasgemisch auch Wasserdampf enthalten sein. Stickstoff ist zur Einstellung der Sauerstoffkonzentration vorhanden, das gleiche gilt für Wasserdampf. Wasserdampf kann ferner zur Abfuhr der Reaktionswärme und als mildes Oxidationsmittel für die Entfernung von kohlenstoffhaltigen Ablagerungen vorhanden sein. Vorzugsweise werden Wasser (als Wasserdampf) und Stickstoff in das Regeneriergasgemisch eingemischt und in den Reaktor eingeleitet. Beim Einleiten von Wasserdampf in den Reaktor zu Beginn der Regeneration wird vorzugsweise ein Volumenanteil von 0-50 %, vorzugsweise 0,5-22 % und noch mehr bevorzugt 1-10 % eingeleitet. Der Anteil an Wasserdampf kann im Laufe der Regeneration erhöht werden. Die Menge an Stickstoff wird so gewählt, dass der Volumenanteil an molekularem Stickstoff im Regeneriergasgemisch zu Beginn der Regeneration 20-99 %, vorzugsweise 50-98 % und noch mehr bevorzugt 70-96 % beträgt. Der Anteil an Stickstoff kann im Laufe der Regeneration erniedrig werden.

Ferner kann das Regeneriergasgemisch Kohlenwasserstoffe enthalten. Diese können zusätzlich zu oder anstelle der Inertgase zugemischt werden. Der Volumenanteil der Kohlenwasserstoffe in dem sauerstoffhaltigen Regeneriergasgemisch ist im Allgemeinen kleiner als 50 %, vorzugsweise kleiner als 10 % und noch mehr bevorzugt kleiner als 2 %. Die Kohlenwasserstoffe können gesättigte und ungesättigte, verzweigte und unverzweigte Kohlenwasserstoffe, wie z. B. Methan, Ethan, Ethen, Acetylen, Propan, Propen, Propin, n-Butan, iso-Butan, n-Buten, iso-Buten, n-Pentan sowie Diene wie 1,3-Butadien und 1,2-Butadien enthalten. Sie enthalten insbesondere Kohlenwasserstoffe, die in Anwesenheit von Sauerstoff unter den Regenerierbedingungen in Gegenwart des Katalysators keine Reaktivität aufweisen.

Während des stabilen Betriebs ist die Verweildauer im Reaktor während der Regeneration in der vorliegenden Erfindung nicht besonders eingeschränkt, aber die Untergrenze beträgt im Allgemeinen 0,3 s oder mehr, vorzugsweise 0,7 s oder mehr und noch mehr bevorzugt 1,0 s oder mehr. Die Obergrenze beträgt 7,0 s oder weniger, vorzugsweise 5,0 s oder weniger und noch mehr bevorzugt 3,5 s oder weniger. Das Verhältnis von Durchfluss an Mischgas bezogen auf das Katalysatorvolumen im Reaktorinnern beträgt 500-8000 $h^{-1}$, vorzugsweise 600-4000 $h^{-1}$ und noch mehr bevorzugt 700-3500 $h^{-1}$.

Vorzugsweise wird der Regenerationsschritt im Wesentlichen den gleichen Drücken durchgeführt wie der Produktionsschritt. Im Allgemeinen liegt der Reaktoreingangsdruck bei < 3 bar (ü), bevorzugt < 2 bar (ü) und besonders bevorzugt < 1,5 (ü) bar. Im Allgemeinen liegt der Reaktorausgangsdruck bei < 2,8 bar (ü), bevorzugt < 1,8 bar (ü) und besonders bevorzugt < 1,3 (ü) bar. Man wählt einen Reaktoreingangsdruck, der ausreicht, die in der Anlage und der nachfolgenden Aufarbeitung vorhandenen Strömungswiderstände zu überwinden. Im Allgemeinen beträgt der Reaktoreingangsdruck mindestens 0,01 bar (ü), bevorzugt mindestens 0,1 bar (ü) und besonders bevorzugt 0,5 bar (ü). Im Allgemeinen beträgt der Reaktorausgangsdruck mindestens 0.01 bar (ü), bevorzugt mindestens 0,1 bar (ü) und besonders bevorzugt 0,2 bar (ü). Der Druckverlust über die gesamte Katalysatorschüttung beträgt im Allgemeinen 0.01 bis 2 bar (ü), bevorzugt 0,1 bis 1,5 bar, besonders bevorzugt 0,4 bis 1,0 bar.

Die Reaktionstemperatur der Regeneration wird durch ein Wärmeaustauschmittel, welches sich um die Reaktionsrohre herum befindet, kontrolliert. Als solche flüssige Wärmeaustauschmittel kommen z. B. Schmelzen von Salzen wie Kaliumnitrat, Kaliumnitrit, Natriumnitrit und/oder Natriumnitrat sowie Schmelzen von Metallen wie Natrium, Quecksilber und Legierungen verschiedener Metalle in Betracht. Aber auch ionische Flüssigkeiten oder Wärmeträgeröle sind einsetzbar. Die Temperatur des Wärmeaustauschmittels liegt zwischen 350 bis 490 °C und bevorzugt zwischen 350 bis 450 °C und besonders bevorzugt zwischen 350 und 420 °C. Die genannten Temperaturen beziehen sich auf die Temperatur des Wärmeaustauschmediums am Einlass für das Wärmeaustauschmedium am Reaktor.

Vorzugsweise liegt die Temperatur im Regenerierzyklus (ii) um bis zu 20 °C, besonders bevorzugt um bis zu 10 °C höher als die Temperatur im Produktionszyklus (i). Bevorzugt liegt die Temperatur im Produktionszyklus oberhalb von 350 °C, besonders bevorzugt oberhalb von 360 °C und insbesondere oberhalb von 365 °C, und beträgt maximal 420 °C. Die genannten Temperaturen beziehen sich auf die Temperatur des Wärmeaustauschmediums am Einlass für das Wärmeaustauschmedium am Reaktor.

Aufarbeitung des Produktgasstroms

Der die oxidative Dehydrierung verlassende Produktgasstrom des Produktionsschritts enthält neben Butadien im Allgemeinen noch nicht umgesetztes n-Butan und iso-Butan, 2-Buten und Wasserdampf. Als Nebenbestandteile enthält er im Allgemeinen Kohlenmonoxid, Kohlendioxid, Sauerstoff, Stickstoff, Methan, Ethan, Ethen, Propan und Propen, gegebenenfalls Wasserstoff sowie sauerstoffhaltige Kohlenwasserstoffe, sogenannte Oxygenate. Im Allgemeinen enthält er nur noch geringe Anteile an 1-Buten und iso-Buten.

Beispielsweise kann der die oxidative Dehydrierung verlassende Produktgasstrom 1 bis 40 Vol-% Butadien, 20 bis 80 Vol-% n-Butan, 0 bis 5 Vol.-% iso-Butan, 0,5 bis 40 Vol-% 2-Buten, 0 bis 5 Vol-% 1-Buten, 0 bis 70 Vol-% Wasserdampf, 0 bis 10 Vol-% leichtsiedende Kohlenwasserstoffe (Methan, Ethan, Ethen, Propan und Propen), 0 bis 40 Vol-% Wasserstoff, 0 bis 30 vol.-% Sauerstoff, 0 bis 70 Vol-% Stickstoff, 0 bis 10 Vol-% Kohlenstoffoxide und 0 bis 10 Vol-% Oxygenate aufweisen. Oxygenate können beispielsweise Formaldehyd, Furan, Essigsäure, Maleinsäureanhydrid, Ameisensäure, Methacrolein, Methacrylsäure, Crotonaldehyd, Crotonsäure, Propionsäure, Acrylsäure, Methylvinylketon, Styrol, Benzaldehyd, Benzoesäure, Phthalsäureanhydrid, Fluorenon, Anthrachinon und Butyraldehyd sein.

Einige der Oxygenate können auf der Katalysatoroberfläche und in der Aufarbeitung weiter oligomerisieren und dehydrieren und dabei Kohlenstoff-, Wasserstoff- und Sauerstoff enthaltende Ablagerungen, im Folgenden als Koks bezeichnet, bilden. Diese Ablagerungen können, zwecks Reinigung und Regeneration, zu Unterbrechungen im Betrieb des Verfahrens führen und sind daher unerwünscht. Typische Koks-Vorläufer umfassen Styrol, Fluorenon und Anthrachinon.

Der Produktgasstrom am Reaktorausgang ist durch eine Temperatur nahe der Temperatur am Ende des Katalysatorbetts charakterisiert. Der Produktgasstrom wird dann auf eine Temperatur von 150 - 400 °C, bevorzugt 160 - 300 °C, besonders bevorzugt 170 - 250 °C gebracht. Es ist möglich, die Leitung, durch die der Produktgasstrom fließt, um die Temperatur im gewünschten Bereich zu halten, zu isolieren, jedoch ist ein Einsatz eines Wärmetauschers bevorzugt. Dieses Wärmetauschersystem ist beliebig, solange mit diesem System die Temperatur des Produktgases auf dem gewünschten Niveau gehalten werden kann. Als Beispiele von Wärmetauschern können Spiralwärmetauscher, Plattenwärmetauscher, Doppelrohrwärmetauscher, Multirohrwärmetauscher, Kessel-Spiralwärmetauscher, Kessel-Mantelwärmetauscher, Flüssigkeit-Flüssigkeit-Kontakt-Wärmetauscher, Luft-Wärmetauscher, Direktkontaktwärmetauscher sowie Rippenrohrwärmetauscher genannt werden. Da, während die Temperatur des Produktgases auf die gewünschte Temperatur eingestellt wird, ein Teil der hochsiedenden Nebenprodukte, die im Produktgas enthalten sind, ausfallen kann, sollte daher das Wärmetauschersystem vorzugsweise zwei oder mehr Wärmetauscher aufweisen. Falls dabei zwei oder mehr vorgesehene Wärmetauscher parallel angeordnet sind, und so eine verteilte Kühlung des gewonnenen Produktgases in den Wärmetauschern ermöglicht wird, nimmt die Menge an hochsiedenden Nebenprodukten, die sich in den Wärmetauschern ablagern, ab, und so kann ihre Betriebsdauer verlängert werden. Als Alternative zu der oben genannten Methode können die zwei oder mehr vorgesehenen Wärmetauscher parallel angeordnet sein. Das Produktgas wird einem oder mehreren, nicht aber allen, Wärmetauscher zugeführt, und nach einer gewissen Betriebsdauer werden diese Wärmetauscher von anderen Wärmetauschern abgelöst. Bei dieser Methode kann die Kühlung fortgesetzt werden, ein Teil der Reaktionswärme zurückgewonnen werden, und parallel dazu können die in einem der Wärmetauscher abgelagerten hochsiedenden Nebenprodukte entfernt werden. Als ein oben genanntes organisches Lösungsmittel kann ein Lösungsmittel, solange es in der Lage ist, die hochsiedenden Nebenprodukte aufzulösen, uneingeschränkt verwendet werden, und als Beispiele dazu können ein aromatisches Kohlenwasserstofflösungsmittel, wie z. B. Toluol, Xylol etc. sowie ein alkalisches wässriges Lösungsmittel, wie z. B. die wässrige Lösung von Natriumhydroxid, verwendet werden.

Enthält der Produktgasstrom mehr als nur geringfügige Spuren Sauerstoff, so kann eine Verfahrensstufe zur Entfernung von Rest-Sauerstoff aus dem Produktgasstrom durchgeführt werden. Der Rest-Sauerstoff kann sich insoweit als störend auswirken, als er in nachgelagerten Verfahrensschritten eine Butadienperoxidbildung hervorrufen kann und als Initiator für Polymerisationsreaktionen wirken kann. Unstabilisiertes 1,3-Butadien kann in Gegenwart von Sauerstoff gefährliche Butadienperoxide bilden. Die Peroxide sind viskose Flüssigkeiten. Ihre Dichte ist höher als die von Butadien. Da sie außerdem nur wenig in flüssigem 1,3-Butadien löslich sind, setzen sie sich auf den Böden von Lagerbehältern ab. Trotz ihrer relativ geringen chemischen Reaktivität sind die Peroxide sehr instabile Verbindungen, die sich bei Temperaturen zwischen 85 und 110 °C spontan zersetzen können. Eine besondere Gefahr besteht in der hohen Schlagempfindlichkeit der Peroxide, die mit der Brisanz eines Sprengstoffes explodieren. Die Gefahr der Polymerbildung ist insbesondere bei der destillativen Abtrennung von Butadien gegeben und kann dort zu Ablagerungen von Polymeren (Bildung von so genanntem "Popcorn") in den Kolonnen führen. Vorzugsweise wird die Sauerstoffentfernung unmittelbar nach der oxidativen Dehydrierung durchgeführt. Im Allgemeinen wird hierzu eine katalytische Verbrennungsstufe durchgeführt, in der Sauerstoff mit in dieser Stufe zugesetztem Wasserstoff in Gegenwart eines Katalysators umgesetzt wird. Hierdurch wird eine Verringerung des Sauerstoffgehalts bis auf geringe Spuren erreicht.

Das Produktgas der $O_2$-Entfernungsstufe wird nun auf ein identisches Temperaturniveau gebracht wie es für den Bereich hinter dem ODH-Reaktor beschrieben worden ist. Die Abkühlung des verdichteten Gases erfolgt mit Wärmetauschern, die beispielsweise als Rohrbündel-, Spiral- oder Plattenwärmetauscher ausgeführt sein können. Die dabei abgeführte Wärme wird bevorzugt zur Wärmeintegration im Verfahren genutzt.

Anschließend können aus dem Produktgasstrom durch Abkühlung ein Großteil der hochsiedenden Nebenkomponenten und des Wassers abgetrennt werden. Diese Abtrennung erfolgt dabei vorzugsweise in einem Quench. Dieser Quench kann aus einer oder mehreren Stufen bestehen. Vorzugsweise wird ein Verfahren eingesetzt, bei dem das Produktgas direkt mit dem Kühlmedium in Kontakt gebracht und dadurch gekühlt wird. Das Kühlmedium ist nicht besonders eingeschränkt, aber vorzugsweise wird Wasser oder eine alkalische wässrige Lösung verwendet. Es wird ein Gasstrom erhalten, in welchem n-Butan, 1-Buten, 2-Butene, Butadien, gegebenenfalls Sauerstoff, Wasserstoff, Wasserdampf, in geringen Mengen Methan, Ethan, Ethen, Propan und Propen, iso-Butan, Kohlenstoffoxide und Inertgase verbleibt. Weiterhin können in diesem Produktgasstrom Spuren von hochsiedenden Komponenten verbleiben, welche im Quench nicht quantitativ abgetrennt wurden.

Anschließend wird der Produktgasstrom aus dem Quench in mindestens einer ersten Kompressionsstufe komprimiert und nachfolgend abgekühlt, wobei mindestens ein Kondensatstrom enthaltend Wasser auskondensiert und ein Gasstrom enthaltend n-Butan, 1-Buten, 2-Butene, Butadien, gegebenenfalls Wasserstoff, Wasserdampf, in geringen Mengen Methan, Ethan, Ethen, Propan und Propen, iso-Butan, Kohlenstoffoxide und Inertgase, gegebenenfalls Sauerstoff und Wasserstoff verbleibt. Die Kompression kann ein- oder mehrstufig erfolgen. Insgesamt wird von einem Druck im Bereich von 1,0 bis 4,0 bar (absolut) auf einen Druck im Bereich von 3,5 bis 20 bar (absolut) komprimiert. Nach jeder Kompressionsstufe folgt eine Abkühlstufe, in der der Gasstrom auf eine Temperatur im Bereich von 15 bis 60 °C abgekühlt wird. Der Kondensatstrom kann somit bei mehrstufiger Kompression auch mehrere Ströme umfassen. Der Kondensatstrom besteht im Allgemeinen zu mindestens 80 Gew.-%, vorzugsweise zu mindestens 90 Gew.-% aus Wasser und enthält daneben in geringem Umfang Leichtsieder, C4-Kohlenwasserstoffe, Oxygenate und Kohlenstoffoxide.

Geeignete Verdichter sind beispielsweise Turbo-, Drehkolben- und Hubkolbenverdichter. Die Verdichter können beispielsweise mit einem Elektromotor, einem Expander oder einer Gas- oder Dampfturbine angetrieben werden. Typische Verdichtungsverhältnisse (Austrittsdruck : Eintrittsdruck) pro Verdichterstufe liegen je nach Bauart zwischen 1,5 und 3,0. Die Abkühlung des verdichteten Gases erfolgt mit Wärmetauschern, die beispielsweise als Rohrbündel-, Spiral- oder Plattenwärmetauscher ausgeführt sein können. Als Kühlmittel kommen in den Wärmetauschern dabei Kühlwasser oder Wärmeträgeröle zum Einsatz. Daneben wird bevorzugt Luftkühlung unter Einsatz von Gebläsen eingesetzt.

Der Butadien, Butene, Butan, Inertgase und gegebenenfalls Kohlenstoffoxide, Sauerstoff, Wasserstoff sowie leicht siedende Kohlenwasserstoffe (Methan, Ethan, Ethen, Propan, Propen) und geringe Mengen von Oxygenaten enthaltende Stoffstrom wird als Ausgangsstrom der weiteren Aufbereitung zugeführt.

Die Abtrennung der leicht siedenden Nebenbestandteile aus dem Produktgasstrom kann durch übliche Trennverfahren wie Destillation, Rektifikation, Membranverfahren, Absorption oder Adsorption erfolgen.

Zur Abtrennung von eventuell im Produktgasstrom enthaltenen Wasserstoff kann das Produktgasgemisch, gegebenenfalls nach erfolgter Kühlung, beispielsweise in einem Wärmetauscher, über eine in der Regel als Rohr ausgebildete Membran geleitet werden, die lediglich für molekularen Wasserstoff durchlässig ist. Der so abgetrennte molekulare Wasserstoff kann bei Bedarf zumindest teilweise in einer Hydrierung eingesetzt oder aber einer sonstigen Verwertung zugeführt werden, beispielsweise zur Erzeugung elektrischer Energie in Brennstoffzellen eingesetzt werden.

Das in dem Produktgasstrom enthaltene Kohlendioxid kann durch $CO_2$-Gaswäsche abgetrennt werden. Der Kohlendioxid-Gaswäsche kann eine gesonderte Verbrennungsstufe vorgeschaltet werden, in der Kohlenmonoxid selektiv zu Kohlendioxid oxidiert wird.

In einer bevorzugten Ausführungsform des Verfahrens werden die nicht kondensierbaren oder leicht siedenden Gasbestandteile wie Wasserstoff, Sauerstoff, Kohlenstoffoxide, die leicht siedenden Kohlenwasserstoffe (Methan, Ethan, Ethen, Propan, Propen) und Inertgas wie gegebenenfalls Stickstoff in einem Absorptions-/Desorptions-Zyklus mittels eines hoch siedenden Absorptionsmittels abgetrennt, wobei ein $C_4$-Produktgasstrom erhalten wird, der im Wesentlichen aus den $C_4$-Kohlenwasserstoffen besteht. Im Allgemeinen besteht der $C_4$-Produktgasstrom zu mindestens 80 Vol-%, bevorzugt zu mindestens 90 Vol-%, besonders bevorzugt zu mindestens 95 Vol-% aus den $C_4$-Kohlenwasserstoffen, im Wesentlichen n-Butan, 2-Buten und Butadien.

Dazu wird in einer Absorptionsstufe der Produktgasstrom nach vorheriger Wasserabtrennung mit einem inerten Absorptionsmittel in Kontakt gebracht und werden die $C_4$-Kohlenwasserstoffe in dem inerten Absorptionsmittel absorbiert, wobei mit $C_4$-Kohlenwasserstoffen beladenes Absorptionsmittel und ein die übrigen Gasbestandteile enthaltendes Abgas erhalten werden. In einer Desorptionsstufe werden die $C_4$-Kohlenwasserstoffe aus dem Absorptionsmittel wieder freigesetzt.

Die Absorptionsstufe kann in jeder beliebigen, dem Fachmann bekannten geeigneten Absorptionskolonne durchgeführt werden. Die Absorption kann durch einfaches Durchleiten des Produktgasstroms durch das Absorptionsmittel erfolgen. Sie kann aber auch in Kolonnen oder in Rotationsabsorbern erfolgen. Dabei kann im Gleichstrom, Gegenstrom oder Kreuzstrom gearbeitet werden. Bevorzugt wird die Absorption im Gegenstrom durchgeführt. Geeignete Absorptionskolonnen sind z. B. Bodenkolonnen mit Glocken-, Zentrifugal- und/oder Siebboden, Kolonnen mit strukturierten Packungen, z. B. Blechpackungen mit einer spezifischen Oberfläche von 100 bis 1000 $m^2/m^3$ wie Mellapak® 250 Y, und Füllkörperkolonnen. Es kommen aber auch Riesel- und Sprühtürme, Graphitblockabsorber, Oberflächenabsorber wie Dickschicht und Dünnschichtabsorber sowie Rotationskolonnen, Tellerwäscher, Kreuzschleierwäscher und Rotationswäscher in Betracht.

In einer Ausführungsform wird einer Absorptionskolonne im unteren Bereich der Butadien, Buten, Butan, und/oder Stickstoff und gegebenenfalls Sauerstoff, Wasserstoff und/oder Kohlendioxid enthaltende Stoffstrom zugeführt. Im oberen Bereich der Absorptionskolonne wird der Lösungsmittel und ggf. Wasser enthaltende Stoffstrom aufgegeben.

In der Absorptionsstufe eingesetzte inerte Absorptionsmittel sind im Allgemeinen hochsiedende unpolare Lösungsmittel, in denen das abzutrennende $C_4$-Kohlenwasserstoff-Gemisch eine deutlich höhere Löslichkeit als die übrigen abzutrennenden Gasbestandteile aufweist. Geeignete Absorptionsmittel sind vergleichsweise unpolare organische Lösungsmittel, beispielsweise aliphatische $C_8$- bis $C_{18}$-Alkane, oder aromatische Kohlenwasserstoffe wie die Mittelölfraktionen aus der Paraffindestillation, Toluol oder Ether mit sperrigen Gruppen, oder Gemische dieser Lösungsmittel, wobei diesen ein polares Lösungsmittel wie 1,2-Dimethylphthalat zugesetzt sein kann. Geeignete Absorptionsmittel sind weiterhin Ester der Benzoesäure und Phthalsäure mit geradkettigen $C_1$-$C_8$-Alkanolen, sowie sogenannte Wärmeträgeröle, wie Biphenyl und Diphenylether, deren Chlorderivate sowie Triarylalkene. Ein geeignetes Absorptionsmittel ist ein Gemisch aus Biphenyl und Diphenylether, bevorzugt in der azeotropen Zusammensetzung, beispielsweise das im Handel erhältliche Diphyl®. Häufig enthält dieses Lösungsmittelgemisch Dimethylphthalat in einer Menge von 0,1 bis 25 Gew.-%.

Geeignete Absorptionsmittel sind Octane, Nonane, Decane, Undecane, Dodecane, Tridecane, Tetradecane, Pentadecane, Hexadecane, Heptadecane und Octadecane oder aus Raffinerieströmen gewonnene Fraktionen, die als Hauptkomponenten die genannten linearen Alkane enthalten.

In einer bevorzugten Ausführungsform wird als Lösungsmittel für die Absorption ein Alkangemisch wie Tetradekan (technischer C14-C17 Schnitt) eingesetzt.

Am Kopf der Absorptionskolonne wird ein Abgasstrom abgezogen, der im wesentlichen Inertgas, Kohlenstoffoxide, gegebenenfalls Butan, Butene, wie 2-Butene und Butadien, ggf. Sauerstoff, Wasserstoff und leicht siedende Kohlenwasserstoffe (zum Beispiel Methan, Ethan, Ethen, Propan, Propen) und Wasserdampf enthält. Dieser Stoffstrom kann teilweise dem ODH-Reaktor oder dem $O_2$-Entfernungsreaktor zugeführt werden. Damit lässt sich zum Beispiel der Eintrittsstrom des ODH-Reaktors auf den gewünschten $C_4$-Kohlenwasserstoffgehalt einstellen.

Der mit $C_4$-Kohlenwasserstoffen beladene Lösungsmittelstrom wird in eine Desorptionskolonne geleitet. Alle dem Fachmann bekannten Kolonneneinbauten sind für diesen Zweck geeignet. In einer Verfahrensvariante wird der Desorptionsschritt durch Entspannung und/oder Erhitzen des beladenen Lösungsmittels durchgeführt. Bevorzugte Verfahrensvariante ist die Zugabe von Strippdampf und/oder die Zufuhr von Frischdampf im Sumpf des Desorbers. Das von $C_4$-Kohlenwasserstoffen abgereicherte Lösungsmittel kann als Gemisch gemeinsam mit dem kondensierten Dampf (Wasser) einer Phasentrennung zugeführt werden, so dass das Wasser vom Lösungsmittel abgeschieden wird. Alle dem Fachmann bekannten Apparate sind hierfür geeignet. Möglich ist zudem die Nutzung des vom Lösungsmittel abgetrennten Wassers zur Erzeugung des Strippdampfes.

Bevorzugt werden 70 bis 100 Gew.-% Lösungsmittel und 0 bis 30 Gew.-% Wasser, besonders bevorzugt 80 bis 100 Gew.-% Lösungsmittel und 0 bis 20 Gew.-% Wasser, insbesondere 85 bis 95 Gew.-% Lösungsmittel und 5 bis 15 Gew.-% Wasser eingesetzt. Das in der Desorptionsstufe regenerierte Absorptionsmittel wird in die Absorptionsstufe zurückgeführt.

Die Abtrennung ist im Allgemeinen nicht ganz vollständig, so dass in dem $C_4$-Produktgasstrom - je nach Art der Abtrennung - noch geringe Mengen oder auch nur Spuren der weiteren Gasbestandteile, insbesondere der schwer siedenden Kohlenwasserstoffe, vorliegen können. Die durch die Abtrennung auch bewirkte Volumenstromverringerung entlastet die nachfolgenden Verfahrensschritte.

Der im Wesentlichen aus n-Butan, Butenen, wie 2-Butenen und Butadien bestehende $C_4$-Produktgasstrom enthält im Allgemeinen 20 bis 80 Vol.-% Butadien, 20 bis 80 Vol.-% n-Butan, 0 bis 10 Vol.-% 1-Buten, und 0 bis 50 Vol.-% 2-Butene, wobei die Gesamtmenge 100 Vol.-% ergibt. Weiterhin können geringe Mengen an iso-Butan enthalten sein.

Der $C_4$-Produktgasstrom kann anschließend durch eine Extraktivdestillation in einen im Wesentlichen aus n-Butan und 2-Buten bestehenden Strom und einen aus Butadien bestehenden Strom getrennt werden. Der im Wesentlichen aus n-Butan und 2-Buten bestehende Strom kann ganz oder teilweise in den $C_4$-Feed des ODH-Reaktors rückgeführt werden. Da die Buten-Isomere dieses Rückführstroms im Wesentlichen aus 2-Butenen bestehen und diese 2-Butene im Allgemeinen langsamer zu Butadien oxidativ dehydriert werden als 1-Buten, kann dieser Rückführstrom vor der Zuführung in den ODH-Reaktor einen katalytischen Isomerisierungsprozess durchlaufen. In diesem katalytischen Prozess kann die Isomerenverteilung entsprechend der im thermodynamischen Gleichgewicht vorliegenden Isomerenverteilung eingestellt werden.

Die Extraktivdestillation kann beispielsweise, wie in "Erdöl und Kohle - Erdgas - Petrochemie", Band 34 (8), Seiten 343 bis 346 oder "Ullmanns Enzyklopädie der Technischen Chemie", Band 9, 4. Auflage 1975, Seiten 1 bis 18 beschrieben, durchgeführt werden. Hierzu wird der $C_4$-Produktgasstrom mit einem Extraktionsmittel, vorzugsweise einem N-Methylpyrrolidon (NMP)/Wasser-Gemisch, in einer Extraktionszone in Kontakt gebracht. Die Extraktionszone ist im Allgemeinen in Form einer Waschkolonne ausgeführt, welche Böden, Füllkörper oder Packungen als Einbauten enthält. Diese weist im Allgemeinen 30 bis 70 theoretische Trennstufen auf, damit eine hinreichend gute Trennwirkung erzielt wird. Vorzugsweise weist die Waschkolonne im Kolonnenkopf eine Rückwaschzone auf. Diese Rückwaschzone dient zur Rückgewinnung des in der Gasphase enthaltenen Extraktionsmittels mit Hilfe eines flüssigen Kohlenwasserstoffrücklaufs, wozu die Kopffraktion zuvor kondensiert wird. Das Massenverhältnis Extraktionsmittel zu $C_4$-Produktgasstrom im Zulauf der Extraktionszone beträgt im Allgemeinen 10 : 1 bis 20 : 1. Die Extraktivdestillation wird vorzugsweise bei einer Sumpftemperatur im Bereich von 100 bis 250 °C, insbesondere bei einer Temperatur im Bereich von 110 bis 210 °C, einer Kopftemperatur im Bereich von 10 bis 100·°C, insbesondere im Bereich von 20 bis 70·°C und einem Druck im Bereich von 1 bis 15 bar, insbesondere im Bereich von 3 bis 8 bar betrieben. Die Extraktivdestillationskolonne weist vorzugsweise 5 bis 70 theoretische Trennstufen auf.

Geeignete Extraktionsmittel sind Butyrolacton, Nitrile wie Acetonitril, Propionitril, Methoxypropionitril, Ketone wie Aceton, Furfural, N-alkylsubstituierte niedere aliphatische Säureamide wie Dimethylformamid, Diethylformamid, Dimethylacetamid, Diethylacetamid, N-Formylmorpholin, N-alkylsubstituierte zyklische Säureamide (Lactame) wie N-Alkylpyrrolidone, insbesondere N-Methylpyrrolidon (NMP). Im Allgemeinen werden alkylsubstituierte niedere aliphatische Säureamide oder N-alkylsubstituierte zyklische Säureamide verwendet. Besonders vorteilhaft sind Dimethylformamid, Acetonitril, Furfural und insbesondere NMP.

Es können jedoch auch Mischungen dieser Extraktionsmittel untereinander, z.B. von NMP und Acetonitril, Mischungen dieser Extraktionsmittel mit Co-Lösungsmitteln und/oder tert.-Butyl-ether, z.B. Methyl-tert.-butylether, Ethyl-tert.-butylether, Propyl-tert.-butylether, n- oder iso-Butyl-tert.-butylether eingesetzt werden. Besonders geeignet ist NMP, bevorzugt in wässriger Lösung, vorzugsweise mit 0 bis 20 Gew.-% Wasser, besonders bevorzugt mit 7 bis 10 Gew.-% Wasser, insbesondere mit 8,3 Gew.-% Wasser.

Der Kopfproduktstrom der Extraktivdestillationskolonne enthält im Wesentlichen Butan und Butene und in geringen Mengen Butadien und wird gasförmig oder flüssig abgezogen. Im Allgemeinen enthält der im Wesentlichen aus n-Butan und 2-Buten bestehende Strom 50 bis 100 Vol-% n-Butan, 0 bis 50 Vol-% 2-Buten und 0 bis 3 Vol-% weitere Bestandteile wie Isobutan, Isobuten, Propan, Propen und $C_5^+$-Kohlenwasserstoffe.

Am Sumpf der Extraktivdestillationskolonne wird ein das Extraktionsmittel, Wasser, Butadien und in geringen Anteilen Butene und Butan enthaltender Stoffstrom gewonnen, der einer Destillationskolonne zugeführt wird. In dieser wird über Kopf oder als Seitenabzug Butadien gewonnen werden. Am Sumpf der Destillationskolonne fällt ein Extraktionsmittel und Wasser enthaltender Stoffstrom an, wobei die Zusammensetzung des Extraktionsmittel und Wasser enthaltenden Stoffstroms der Zusammensetzung entspricht, wie sie der Extraktion zugegeben wird. Der Extraktionsmittel und Wasser enthaltende Stoffstrom wird bevorzugt in die Extraktivdestillation zurückgeleitet.

Die Extraktionslösung wird in eine Desorptionszone überführt, wobei aus der Extraktionslösung das Butadien desorbiert wird. Die Desorptionszone kann beispielsweise in Form einer Waschkolonne ausgeführt sein, die 2 bis 30, bevorzugt 5 bis 20 theoretische Stufen und gegebenenfalls eine Rückwaschzone mit beispielsweise 4 theoretischen Stufen aufweist. Diese Rückwaschzone dient zur Rückgewinnung des in der Gasphase enthaltenen Extraktionsmittels mit Hilfe eines flüssigen Kohlenwasserstoffrücklaufs, wozu die Kopffraktion zuvor kondensiert wird. Als Einbauten sind Packungen, Böden oder Füllkörper vorgesehen. Die Destillation wird vorzugsweise bei einer Sumpftemperatur im Bereich von 100 bis 300 °C, insbesondere im Bereich von 150 bis 200 °C und einer Kopftemperatur im Bereich von 0 bis 70 °C, insbesondere im Bereich von 10 bis 50 °C durchgeführt. Der Druck in der Destillationskolonne liegt dabei vorzugsweise im Bereich von 1 bis 10 bar. Im Allgemeinen herrscht in der Desorptionszone gegenüber der Extraktionszone verminderter Druck und/oder eine erhöhte Temperatur.

Der am Kolonnenkopf gewonnene Wertproduktstrom enthält im Allgemeinen 90 bis 100 Vol-% Butadien, 0 bis 10 Vol.-% 2-Buten und 0 bis 10 Vol.-% n-Butan und iso-Butan. Zur weiteren Aufreinigung des Butadiens kann eine weitere Destillation nach dem Stand der Technik durchgeführt werden.

Die Erfindung wird durch die nachstehenden Beispiele näher erläutert.

Die in den Beispielen berechneten Größen Umsatz (X) und Selektivität (S) wurden wie folgt bestimmt:

$$X = \frac{mol(Butene_{ein}) - mol(Butene_{aus})}{mol(Butene_{ein})}$$

$$S = \frac{mol(Butadien_{aus}) - mol(Butadien_{ein})}{mol(Butene_{ein}) - mol(Butene_{aus})}$$

wobei mol($XXX_{ein}$) die Stoffmenge der Komponente XXX am Reaktoreingang ist, mol($XXX_{aus}$) die Stoffmenge der

Komponente XXX am Reaktorausgang ist und Butene die Summe aus 1-Buten, cis-2-Buten, trans-2-Buten und iso-Buten darstellt.

**Beispiele**

Katalsatorherstellung

Beispiel 1

Es wurden 2 Lösungen A und B hergestellt.

Lösung A:

In einem 10 l-Edelstahltopf wurden 3200 g Wasser vorgelegt. Unter Rühren mittels eines Ankerrührers wurden 5,2 g einer KOH-Lösung (32 Gew. % KOH) zum vorgelegten Wasser zugegeben. Die Lösung wurde auf 60 °C erwärmt. Nun wurden 1066 g einer Ammoniumheptamolybdatlösung ($(NH_4)_6Mo_7O_{24}$*4 $H_2O$, 54 Gew. % Mo) portionsweise über einen Zeitraum von 10 Minuten zugegeben. Die erhaltene Suspension wurde noch 10 Minuten nachgerührt.

Lösung B:

In einem 5 l-Edelstahltopf wurden 1771 g einer Kobalt(II)nitratlösung (12,3 Gew.-% Co) vorgelegt und unter Rühren (Ankerrührer) auf 60 °C erhitzt. Nun wurden 645 g einer Eisen(III)nitratlösung (13,7 Gew.-% Fe) über einen Zeitraum von 10 Minuten portionsweise unter Aufrechterhaltung der Temperatur zugegeben. Die entstandene Lösung wurde 10 min nachgerührt. Nun wurden 619 g einer Bismutnitratlösung (10,7 Gew.-% Bi) unter Aufrechterhaltung der Temperatur zugegeben. Nach weiteren 10 Minuten Nachrühren wurden 109 g Chrom(III)nitrat portionsweise fest zugegeben und die entstandene dunkelrote Lösung 10 min weitergerührt.

Unter Beibehaltung der 60 °C wurde innerhalb von 15 min die Lösung B zur Lösung A mittels Schlauchpumpe zu gepumpt. Während der Zugabe und danach wurde mittels eines Intensivmischers (Ultra-Turrax) gerührt. Nach vollendeter Zugabe wurde noch 5 Minuten weitergerührt. Danach wurden 93.8 g einer $SiO_2$ Suspension (Ludox; $SiO_2$ ca. 49% , Fa. Grace) zugegeben und weitere 5 Minuten gerührt.

Die erhaltene Suspension wurde in einem Sprühturm der Fa. NIRO (Sprühkopf-Nr. FOA1, Drehzahl 25000 U/min) über einen Zeitraum von 1,5 h sprühgetrocknet. Dabei wurde die Vorlagetemperatur bei 60 °C gehalten. Die Gaseingangstemperatur des Sprühturmes betrug 300 °C, die Gasausgangstemperatur 110 °C. Das erhaltene Pulver hatte eine Partikelgröße ($d_{-50}$) kleiner 40 μm.

Das erhaltene Pulver wurde mit 1 Gew.-% Graphit vermischt, zweimal mit 9 bar Pressdruck kompaktiert und durch ein Sieb mit Maschenweite 0.8 mm zerkleinert. Der Split wurde wiederum mit 2 Gew.-% Graphit vermengt und die Mischung mit einer Kilian S100

Tablettenpresse in Ringe 5 x 3 x 2 mm (Außendurchmesser x Länge x Innendurchmesser) gepresst.

Der erhaltene Katalysatorvorläufer wurde chargenweise (500 g) in einem Umluftofen der Firma Heraeus, DE (Typ K, *750/2* S, Innenvolumen 55 l) kalziniert. Folgendes Programm wurde dafür verwendet:

- Aufheizen in 72 Minuten auf 130 °C, 72 Minuten halten
- Aufheizen in 36 Minuten auf 190 °C, 72 Minuten halten
- Aufheizen in 36 Minuten auf 220 °C, 72 Minuten halten
- Aufheizen in 36 Minuten auf 265 °C, 72 Minuten halten
- Aufheizen in 93 Minuten auf 380 °C, 187 Minuten halten
- Aufheizen in 93 Minuten auf 430 °C, 187 Minuten halten
- Aufheizen in 93 Minuten auf 490 °C, 467 Minuten halten

Nach der Kalzination wurden der Katalysator der berechneten Stöchiometrie $Mo_{12}Co_7Fe_3Bi_{0.6}K_{0.08}Cr_{0.5}Si_{1.6}O_x$ erhalten.

Beispiel 2

Die kalzinierten Ringe aus Beispiel 1 wurden zu einem Pulver vermahlen.

Mit dieser Vorläufermasse wurden Trägerkörper (Steatitringe mit Abmessungen 5 x 3 x 2 mm (Außendurchmesser x Länge x Innendurchmesser) beschichtet. Dazu wurden 1054 g des Trägers in einer Dragiertrommel (2 l Innenvolumen, Neigungswinkel der Trommelmittelachse gegen die Horizontale = 30°) vorgelegt. Die Trommel wurde in Rotation versetzt (25 U/min). Über eine mit Druckluft betriebenen Zerstäuberdüse wurden über ca. 30 Minuten hinweg ca. 60 ml flüssiges Bindemittel (Mischung Glycerin : Wasser 1:3) auf den Träger gesprüht (Sprühluft 500 NI/h). Die Düse war dabei derart installiert, dass der Sprühkegel die in der Trommel beförderten Trägerkörper in der oberen Hälfte der Abrollstrecke benetzte. 191 g der feinpulvrigen Vorläufermasse des gemahlenen Katalysators wurden über eine Pulverschnecke in die Trommel eingetragen, wobei der Punkt der Pulverzugabe innerhalb der Abrollstrecke, aber unterhalb des Sprühkegels lag. Die Pulverzugabe wurde dabei so dosiert, dass eine gleichmäßige Verteilung des Pulvers auf der Oberfläche entstand. Nach Abschluss der Beschichtung wurde der entstandene Schalenkatalysator aus Vorläufermasse und dem Trägerkörper in einem Trockenschrank bei 300 °C für 4 Stunden getrocknet.

Dehydrierungsversuche

In einem Screening-Reaktor wurden Dehydrierungsversuche durchgeführt. Der Screening-Reaktor war ein Salzbadreaktor mit einer Länge von 120 cm und einem Innendurchmesser von 14,9 mm und einer Thermohülse mit einem Außendurchmesser von 3,17 mm. In der Thermohülse befand sich ein Mehrfachthermoelement mit 7 Messstellen. Die untersten 4 Messstellen hatten einen Abstand von 10 cm und die obersten 4 Messstellen einen Abstand von 5 cm.

Butan sowie 1-Buten wurden bei circa 10 bar flüssig durch einen Koriolis-Flussmesser dosiert, in einem statischen Mischer vermischt und anschließend in einer beheizten Verdampferstrecke entspannt und verdampft. Dieses Gas wurde nun mit Stickstoff gemischt und in einem Vorheizer mit einer Steatitschüttung geleitet. Wasser wurde flüssig dosiert und in einer Verdampferwendel in einem Luftstrom verdampft. Das Luft/Wasserdampfgemsich wurde im unteren Bereich des Vorheizers mit dem N2/Raffinat-II/Butan-Gemisch vereinigt. Das komplett vermischte Eduktgas wurde dann dem Reaktor zugeführt, wobei ein Analysenstrom für die online-GC-Messung abgezogen werden kann. Aus dem Produktgas, welches den Reaktor verlässt, wird ebenfalls ein Analysenstrom abgezogen, welcher per online-GC-Messung oder mit Hilfe eines IR-Analysators auf den Volumenanteil an CO und CO2 analysiert werden kann. Ein Druckregelventil schließt sich hinter dem Abzweig der Analysenleitung an, welches das Druckniveau des Reaktors einstellt.

Beispiel 3

120 g Steatitringe der Geometrie von 5 x 3 x 2 mm (Außendurchmesser x Länge x Innendurchmesser), die gemäß Beispiel 2 mit 15 Gew.% Aktivmasse eines Katalysators der Zusammensetzung $Mo_{12}Co_7Fe_3Cr_{0,5}K_{0,08}Bi_{0,6}Si_{1,6}$ beschichtet sind, werden in einen Salzbadreaktor mit einem Innendurchmesser von 14,9 mm eingebaut. Der Reaktor wird mit 200 NL/h eines Reaktionsgases der Zusammensetzung 8 Vol.-% Buten, 2 Vol.-% Butan, 7,5% Sauerstoff, 14 Vol.-% Wasser, 67,5 Vol.-% Stickstoff bei einer Salzbadtemperatur von 384 °C für 20 Stunden betrieben. Die Produktgase werden mittels GC analysiert.

Nach jedem Produktionsschritt wird ein Gemisch von 10% Sauerstoff, 80% Stickstoff und 10% Wasser für 15 Minuten bei gleicher Temperatur über den Katalysator geleitet. Die entstehenden Kohlenoxide werden mittels eines IR-Messgeräts aufgezeichnet. Insgesamt werden 5 Zyklen aus Produktions- und Regenerierungsschritten durchlaufen.

Die durchschnittlichen Umsatz- und Selektivitätsdaten während der jeweiligen Produktionsschritte 1 bis 5 sind in Tabelle 1 aufgeführt.

Tabelle 1

| Zyklus Nummer | 1 | 2 | 3 | 4 | 5 |
|---|---|---|---|---|---|
| Umsatz (Buten) | 82.2% | 81.8% | 81.8% | 81.3% | 81.6% |
| Selektivität (Butadien) | 87.8% | 86.7% | 85.8% | 86.5% | 86.8% |

Beispiel 4

44 g Tabletten der Geometrie 5 x 3 x 2 mm Ringe (Außendurchmesser x Länge x Innendurchmesser) und der Zusammensetzung $Mo_{12}Co_7Fe_3Cr_{0,5}K_{0,08}Bi_{0,6}Si_{1,6}$ gemäß Beispiel 1 werden mit 88 g Steatitringen gleicher Geometrie gemischt und in einen Salzbadreaktor mit 14,9 mm Innendurchmesser gefüllt. Der Reaktor wird mit 200 NL/h eines Reaktionsgases der Zusammensetzung 8 Vol.-% Buten, 2 Vol.-% Butan, 7,5 Vol.-% Sauerstoff, 15 Vol.-% Wasser, 67,5 Vol.-% Stickstoff bei einer Salzbadtemperatur von 348 °C für 20 Stunden betrieben. Die Produktgase werden mittels GC analysiert.

Nach jedem Produktionsschritt wird ein Gemisch von 10 % Sauerstoff, 80 % Stickstoff und 10 % Wasser für 30 Minuten bei gleicher Temperatur über den Katalysator geleitet. Die entstehenden Kohlenoxide werden mittels eines IR-Messgeräts aufgezeichnet. Insgesamt werden 5 Zyklen aus Produktions- und Regenerierungsschritten durchlaufen.

Die durchschnittlichen Umsatz- und Selektivitätsdaten während der jeweiligen Produktionsschritte 1 bis 5 sind in Tabelle 2 aufgeführt.

Tabelle 2

| Zyklus Nummer | 1 | 2 | 3 | 4 | 5 |
|---|---|---|---|---|---|
| Umsatz (Buten) | 80.5% | 79.5% | 79.5% | 78.0% | 77.8% |
| Selektivität (Butadien) | 88.7% | 88.4% | 87.6% | 87.2% | 84.0% |

Beispiel 5

120 g Steatitringe der Geometrie von 5 x 3 x 2 mm (Außendurchmesser x Länge x Innendurchmesser), die gemäß Beispiel 2 mit 15 Gew.-% Aktivmasse eines Katalysators der Zusammensetzung $Mo_{12}Co_7Fe_3Cr_{0,5}K_{0,08}Bi_{0,6}Si_{1,6}$ beschichtet sind, werden in einen Salzbadreaktor mit 14,9 mm Innendurchmesser gefüllt. Der Reaktor wird mit 70 NL/h eines Reaktionsgases der Zusammensetzung 8 Vol.-% 1-Buten, 2 Vol.-% Butan, 7,5 Vol.-% Sauerstoff, 10 Vol.-% Wasser, 72,5 Vol.-% Stickstoff bei einer Salzbadtemperatur von 348 °C für 20 Stunden betrieben. Die Produktgase werden mittels GC analysiert.

Nach jedem Produktionsschritt wird ein Gemisch von 10 % Sauerstoff, 80 % Stickstoff und 10 % Wasser für 30 Minuten bei gleicher Temperatur über den Katalysator geleitet. Die entstehenden Kohlenoxide werden mittels eines IR-Messgeräts aufgezeichnet. Insgesamt werden 5 Zyklen aus Produktions- und Regenerierungsschritten durchlaufen.

Die durchschnittlichen Umsatz- und Selektivitätsdaten während der jeweiligen Produktionsschritte 1 bis 5 sind in Tabelle 3 aufgeführt.

Tabelle 3

| Zyklus Nummer | 1 | 2 | 3 | 4 | 5 |
|---|---|---|---|---|---|
| Umsatz (Buten) | 85,3 | 83,6 | 81,4 | 81,0 | 78,1 |
| Selektivität (Butadien) | 76,4 | 69,5 | 82,9 | 80,3 | 73,9 |

Der Umsatz fällt mit dem Katalysator, der unterhalb von 350 °C betrieben wird, kontinuierlich ab, bleibt aber mit dem Katalysator, der oberhalb von 350 °C betrieben wird, konstant.

**Patentansprüche**

1.   Verfahren zur oxidativen Dehydrierung von n-Butenen zu Butadien, umfassend mindestens zwei Produktionsschritte (i) und mindestens einen Regenerierschritt (ii), bei dem

(i) in einem Produktionsschritt ein n-Butene enthaltendes Ausgangsgasgemisch mit einem Sauerstoff enthaltenden Gas gemischt und in einem Festbettreaktor mit einem in einem Katalysatorfestbett angeordneten Multimetalloxid-Katalysator, enthaltend mindestens Molybdän und ein weiteres Metall, in Kontakt gebracht wird, und

(ii) in einem Regenerierschritt der Multimetalloxid-Katalysator durch Überleiten eines sauerstoffhaltigen Regeneriergasgemischs über das Katalysatorfestbett und Abbrennen des auf dem Katalysator abgeschiedenen Kohlenstoffs regeneriert wird,

wobei zwischen zwei Produktionsschritten (i) ein Regenerierschritt (ii) durchgeführt wird, **dadurch gekennzeichnet, dass** die mindestens zwei Produktionsschritte (i) bei einer Temperatur von mindestens 350 °C und der mindestens eine Regenerierschritt (ii) bei einer Temperatur durchgeführt wird, die höchstens 50 °C über der Temperatur, bei der der vorausgegangene Produktionsschritt (i) durchgeführt wurde, liegt.

2.   Verfahren nach Anspruch 1, **dadurch gekennzeichnet, dass** die mindestens zwei Produktionsschritte bei einer Temperatur von mindestens 365 °C durchgeführt werden.

3.   Verfahren nach Anspruch 1 oder 2, **dadurch gekennzeichnet, dass** der mindestens eine Regenerierschritt (ii) bei einer Temperatur durchgeführt wird, die höchstens 20 °C über der Temperatur, bei der die mindestens zwei Produktionsschritte (i) durchgeführt werden, liegt.

4.  Verfahren nach Anspruch 3, **dadurch gekennzeichnet, dass** der mindestens eine Regenerierschritt (ii) bei einer Temperatur durchgeführt wird, der um höchstens 10 °C über der Temperatur, bei der die mindestens zwei Produktionsschritte (i) durchgeführt werden, liegt.

5.  Verfahren nach einem der Ansprüche 1 bis 4, **dadurch gekennzeichnet, dass** das sauerstoffhaltige Regeneriergasgemisch 0,5 bis 22 Vol.-% Sauerstoff enthält.

6.  Verfahren nach einem der Ansprüche 1 bis 5, **dadurch gekennzeichnet, dass** das sauerstoffhaltige Regeneriergasgemisch 0 bis 30 Vol.-% Wasserdampf enthält.

7.  Verfahren nach einem der Ansprüche 1 bis 6, **dadurch gekennzeichnet, dass** die mindestens zwei Produktionsschritte bei einer Temperatur von höchstens 420 °C durchgeführt werden.

8.  Verfahren nach einem der Ansprüche 1 bis 7, **dadurch gekennzeichnet, dass** ein Regenerierschritt (ii) durchgeführt wird, bevor der Umsatzverlust bei konstanter Temperatur mehr als 25% beträgt.

9.  Verfahren nach einem der Ansprüche 1 bis 5, **dadurch gekennzeichnet, dass** pro Regenerierschritt (ii) 5 bis 50 Gew.-% des auf dem Katalysator abgeschiedenen Kohlenstoffs abgebrannt werden.

10. Verfahren nach Anspruch 9, **dadurch gekennzeichnet, dass** pro Regenerierschritt (ii) 10 bis 25 Gew.-% des auf dem Katalysator abgeschiedenen Kohlenstoffs abgebrannt werden.

11. Verfahren nach einem der Ansprüche 1 bis 10, **dadurch gekennzeichnet, dass** das Molybdän und mindestens ein weiteres Metall enthaltende Multimetalloxid die allgemeinen Formel (I)

$$Mo_{12}Bi_aFe_bCo_cNi_dCr_eX^1_fX^2_gO_x \qquad (I)$$

in der die Variablen nachfolgende Bedeutung aufweisen:

X1 = W, Sn, Mn, La, Ce, Ge, Ti, Zr, Hf, Nb, P, Si, Sb, Al, Cd und/oder Mg;
X2 = Li, Na, K, Cs und/oder Rb,
a = 0,1 bis 7, vorzugsweise 0,3 bis 1,5;
b = 0 bis 5, vorzugsweise 2 bis 4;
c = 0 bis 10, vorzugsweise 3 bis 10;
d = 0 bis 10;
e = 0 bis 5, vorzugsweise 0,1 bis 2;
f = 0 bis 24, vorzugsweise 0,1 bis 2;
g = 0 bis 2, vorzugsweise 0,01 bis 1; und
x = eine Zahl, die durch die Wertigkeit und Häufigkeit der von Sauerstoff verschiedenen Elemente in (I) bestimmt wird,

aufweist.

**Claims**

1.  A process for the oxidative dehydrogenation of n-butenes to butadiene, which comprises at least two production steps (i) and at least one regeneration step (ii), in which

    (i) in one production step, a starting gas mixture comprising n-butenes is mixed with an oxygen-comprising gas and brought into contact with a multimetal oxide catalyst which comprises at least molybdenum and a further metal and is arranged in a fixed catalyst bed in a fixed-bed reactor,
    and
    (ii) in a regeneration step, the multimetal oxide catalyst is regenerated by passing an oxygen-comprising regeneration gas mixture over the fixed catalyst bed and burning off the carbon deposited on the catalyst,

    where a regeneration step (ii) is carried out between two production steps (i),
    wherein the at least two production steps (i) are carried out at a temperature of at least 350°C and the at least one regeneration step (ii) is carried out at a temperature which is not more than 50°C above the temperature at which the preceding production step (i) was carried out.

2. The process according to claim 1, wherein the at least two production steps are carried out at a temperature of at least 365°C.

3. The process according to claim 1 or 2, wherein the at least one regeneration step (ii) is carried out at a temperature which is not more than 20°C above the temperature at which the at least two production steps (i) are carried out.

4. The process according to claim 3, wherein the at least one regeneration step (ii) is carried out at a temperature which is not more than 10°C above the temperature at which the at least two production steps (i) are carried out.

5. The process according to any of claims 1 to 4, wherein the oxygen-comprising regeneration gas mixture comprises from 0.5 to 22% by volume of oxygen.

6. The process according to any of claims 1 to 5, wherein the oxygen-comprising regeneration gas mixture comprises from 0 to 30% by volume of steam.

7. The process according to any of claims 1 to 6, wherein the at least two production steps are carried out at a temperature of not more than 420°C.

8. The process according to any of claims 1 to 7, wherein a regeneration step (ii) is carried out before the decrease in conversion at constant temperature is more than 25%.

9. The process according to any of claims 1 to 5, wherein from 5 to 50% by weight of the carbon deposited on the catalyst is burnt off per regeneration step (ii).

10. The process according to claim 9, wherein from 10 to 25% by weight of the carbon deposited on the catalyst is burnt off per regeneration step (ii).

11. The process according to any of claims 1 to 10, wherein the multimetal oxide which comprises molybdenum and at least one further metal has the general formula (I)

$$Mo_{12}Bi_aFe_bCo_cNi_dCr_eX^1_fX^2_gO_x \qquad (I),$$

where the variables have the following meanings:

$X^1$ = W, Sn, Mn, La, Ce, Ge, Ti, Zr, Hf, Nb, P, Si, Sb, Al, Cd and/or Mg;
$X^2$ = Li, Na, K, Cs and/or Rb,
a = 0.1 to 7, preferably from 0.3 to 1.5;
b = 0 to 5, preferably from 2 to 4;
c = 0 to 10, preferably from 3 to 10;
d = 0 to 10;
e = 0 to 5, preferably from 0.1 to 2;
f = 0 to 24, preferably from 0.1 to 2;
g = 0 to 2, preferably from 0.01 to 1; and
x = is a number determined by the valence and abundance of the elements other than oxygen in (I).

**Revendications**

1. Procédé pour la déshydrogénation oxydative de n-butène en butadiène, comprenant au moins deux étapes de production (i) et au moins une étape de régénération (ii), dans lequel

(i) dans une étape de production on mélange un mélange de gaz de départ contenant des n-butènes avec un gaz contenant de l'oxygène et, dans un réacteur à lit fixe, on le met en contact avec un catalyseur à oxyde multimétallique, disposé dans un lit fixe de catalyseur, contenant au moins du molybdène et un autre métal, et

(ii) dans une étape de régénération on régénère le catalyseur à oxyde multimétallique en faisant passer un mélange de gaz de régénération contenant de l'oxygène sur le lit fixe de catalyseur et brûlage du carbone déposé sur le catalyseur,

une étape de régénération (ii) étant effectuée entre deux étapes de production (i),
**caractérisé en ce qu'**on effectue lesdites au moins deux étapes de production (i) à une température d'au moins 350 °C et ladite au moins une étape de régénération (ii) à une température qui est supérieure d'au maximum 50 °C à la température à laquelle a été effectuée l'étape de production (i) précédente.

2.  Procédé selon la revendication 1, **caractérisé en ce qu'**on effectue lesdites au moins deux étapes de production à une température d'au moins 365 °C.

3.  Procédé selon la revendication 1 ou 2, **caractérisé en ce qu'**on effectue ladite au moins une étape de régénération (ii) à une température qui est supérieure d'au maximum 20 °C à la température à laquelle sont effectuées lesdites au moins deux étapes de production (i).

4.  Procédé selon la revendication 3, **caractérisé en ce qu'**on effectue ladite au moins une étape de régénération (ii) à une température qui est supérieure d'au maximum 10 °C à la température à laquelle sont effectuées lesdites au moins deux étapes de production (i).

5.  Procédé selon l'une quelconque des revendications 1 à 4, **caractérisé en ce que** le mélange de gaz de régénération contenant de l'oxygène contient de 0,5 à 22 % en volume d'oxygène.

6.  Procédé selon l'une quelconque des revendications 1 à 5, **caractérisé en ce que** le mélange de gaz de régénération contenant de l'oxygène contient de 0 à 30 % en volume de vapeur d'eau.

7.  Procédé selon l'une quelconque des revendications 1 à 6, **caractérisé en ce que** lesdites au moins deux étapes de production sont effectuées à une température d'au maximum 420 °C.

8.  Procédé selon l'une quelconque des revendications 1 à 7, **caractérisé en ce qu'**on effectue une étape de régénération (ii) avant que la perte de conversion à température constante soit de plus de 25 %.

9.  Procédé selon l'une quelconque des revendications 1 à 5, **caractérisé en ce que** 5 à 50 % en poids du carbone déposé sur le catalyseur sont brûlés par étape de régénération (ii).

10. Procédé selon la revendication 9, **caractérisé en ce que** 10 à 25 % en poids du carbone déposé sur le catalyseur sont brûlés par étape de régénération (ii).

11. Procédé selon l'une quelconque des revendications 1 à 10, **caractérisé en ce que** l'oxyde multimétallique contenant du molybdène et au moins un autre métal présente la formule générale (I)

$$Mo_{12}Bi_aFe_bCo_cNi_dCr_eX^1_fX^2_gO_x \qquad (I)$$

dans laquelle les variables ont la signification suivante :

$X1$ = W, Sn, Mn, La, Ce, Ge, Ti, Zr, Hf, Nb, P, Si, Sb, Al, Cd et/ou Mg ;
$X2$ = Li, Na, K, Cs et/ou Rb ;
$a$ = 0,1 à 7, de préférence 0,3 à 1,5 ;
$b$ = 0 à 5, de préférence 2 à 4 ;
$c$ = 0 à 10, de préférence 3 à 10 ;
$d$ = 0 à 10 ;
$e$ = 0 à 5, de préférence 0,1 à 2 ;
$f$ = 0 à 24, de préférence 0,1 à 2 ;
$g$ = 0 à 2, de préférence 0,01 à 1 ; et
$x$ = un nombre qui est déterminé par la valence et la fréquence des éléments différents de l'oxygène dans (I).

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2009124945 A
- WO 2010137595 A
- JP 60058928 A
- WO 2005047226 A
- US 3911039 A
- DE 2530959 A
- DE 2447825 A
- US 4423281 A
- US 4336409 A
- DE 2600128 A
- DE 2440329 A

- DE 10046957 A
- WO 0224620 A
- US 20060205978 A
- EP 0714700 A
- DE 1642921 A
- DE 2106796 A
- DE 2626887 A
- DE 2909670 A
- DE 2909671 A
- EP 714700 A

**In der Beschreibung aufgeführte Nicht-Patentliteratur**

- *Erdöl und Kohle - Erdgas - Petrochemie,* vol. 34 (8), 343-346

- Ullmanns Enzyklopädie der Technischen Chemie. 1975, vol. 9, 1-18